(19) Europäisches Patentamt
European Patent Office
Office européen des brevets

(11) EP 0 615 756 B1

(12) EUROPEAN PATENT SPECIFICATION

(45) Date of publication and mention of the grant of the patent:
**27.06.2001 Bulletin 2001/26**

(51) Int Cl.[7]: **A61K 31/485**, A61K 9/08

(21) Application number: **94102571.0**

(22) Date of filing: **21.02.1994**

# (54) Nalbuphine esters and long-acting pharmaceutical compositions containing them

Nalbuphinester und diese enthaltende Arzneimittel mit verzögerter Wirkstoffabgabe

Esters de nalbuphine, et compositions pharmaceutiques à action prolongée les contenant

(84) Designated Contracting States:
**CH DE FR GB LI**

(30) Priority: **15.03.1993 JP 5384093**
**02.12.1993 US 161257**

(43) Date of publication of application:
**21.09.1994 Bulletin 1994/38**

(73) Proprietor: **NATIONAL SCIENCE COUNCIL**
**Taipei (TW)**

(72) Inventors:
- **Oliver, Yoa-Pu Hu**
  **Taipei (TW)**
- **Wang, Jhi-Joung**
  **Taipei (TW)**
- **Ho, Shung-Tai**
  **Taipei (TW)**

(74) Representative: **Jones, Stephen Anthony et al**
**AdamsonJones**
**Broadway Business Centre**
**32a Stoney Street**
**Nottingham NG1 1LL (GB)**

(56) References cited:
**EP-A- 0 170 090** **EP-A- 0 250 796**
**WO-A-82/03768** **US-A- 4 722 928**

- **CHEMICAL ABSTRACTS, vol. 110, no. 2, 9 January 1989, Columbus, Ohio, US; abstract no. 13439q, & PHARM.RES. vol. 5, no. 9 , 1988 pages 615 - 618 M.A.HUSSAIN ET AL. 'IMPROVED BUCCAL DELIVERY OF OPIOID ANALGESICS AND ANTAGONISTS WITH BITTERLESS PRODRUGS'**
- **CHEMICAL ABSTRACTS, vol. 107, no. 18, 2 November 1987, Columbus, Ohio, US; abstract no. 161526u, & INT.J.PHARM. vol. 38, no. 1-3 , 1987 pages 199 - 209 B.J.AUNGST ET AL. 'PRODRUGS FOR IMPROVED ORAL NALBUPHINE BIOAVAILABILITY:INTER-SPECIES DIFFERENCES IN THE DISPOSITION OF NALBUPHINE AND ITS ACETYLSALICYLATE AND ANTHRANILATE ESTERS'**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Background of the invention

This invention is related to novel long acting analgesic Nalbuphine prodrugs, and to other pharmaceutical compositions. An ideal analgesic should be characterized by a short onset tine, be long acting, potent, cause no addiction, no cardic or respiratory inhibition and fever other adverse effects. For the relief of pain, local anethetics like xylocaine or bupivacaine can only be applay to a restricted areas. In addition local anesthetics are short acting. Even though they are given intracerebroventricularly the duration hardly exceeds 6 hours. Thereof, for severe and deep pain caused by cardiac, pulmonary, abdominal, osteopathia, and obstetrical surgery or severe burn injury and terminal stages of cancer, local anesthetics are not applicable.

Nonnarcotic analgesics, such as aspirin and acetaminophen, relieve pain of only low intensity, particularly the pain of headache or toothache. They can not do much to help severe pain, either. For pain of high intensity and widespread in origin, narcotic analgesics are certainly indicated. Morphine, meperidine, fentanyl interacting with specific receptors (i.e. mu receptor) in the CNS belong to narcotic analgesics and exhibit potent analgesic activity. Hayes, A. G. et al. (Br. J. Pharmacol. vol. 79, 731, 1983) have reported that all of the narcotic analgesics share the same disadvantages with respect to addiction, addition and respiratory inhibition. For long term use, addiction and addition are the most unwanted problems. Severe respiratory depression and death often occur seen in patients with poor respiratory function post cardiac or chest surgery. In addition, the duration of narcotic analgesics effects are somewhat short in clinical use. In order to maintain an analgesic effect, the dosing interval needs to be set at 3-5 hours. Even though the agent is given via a spinal marrow, the duration could not be longer than 48 hours. In the case of using larger amounts of narcotic analgesic (e.g. morphine, 0.5-1.0 mg/dose) to prolong the effect, as described in the literature published by Baxter,A.D. et al. (Can.J. Anesth. vol. 36, 503, 1989), fatal respiratory depression is likely to occur.

Figure (2)

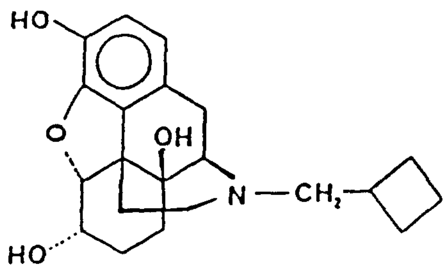

NALBUPHINE

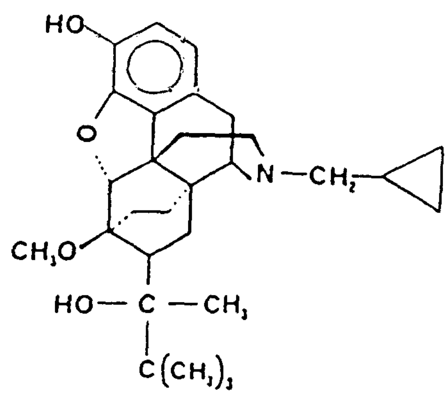

BUPRENORPHINE

Recently new type of opiods so-called narcotic agonist-antagonist analgesics have been developed. Typical ones such as buprenorphine, nalbuphine are shown in Figure 2 there exhibit a dual action of agonism and antagonism on opiods receptors as reported by Schmidt, W.K. et al. (Drug Alcohol Depend, vol. 14, 339, 1985). For instance, nalbuphine is the antagonist of mu receptor and at the same time the agonist of the kappa receptor as well. After a six-month treatment period, nalbuphine showed no significant addiction and addition with only slight respiratory inhibition. Due to this special pharmacologic property, the adverse effects associated with narcotic analgesics are greatly improved. They decrease the incidence of addiction and addition and diminish the inhibitory effect on the respiratory system. Shafer, S.L. et al. have investigated the analgesic potency of this new type of narcotic analgesic, and have found that when compared with more traditional types, to elicit the same analgesic effect, the dose needed for morphine is 10 mg, for buprenorphine 0.3 mg, for nalbuphine 10 mg and for butorphanol 2 mg (Anesthesiology, vol.74, 53, 1991). According to the paper by Schmidt, W.K. et al. (1985), it was reported that nalbuphine is the one most widely used and has excellent therapeutic efficacy. After continuous use of nalbuphine for 6 months, no significant addiction and addition was found. This new type of analgesic only slightly exhibits respiratory inhibition. In clinical use, nalbuphine is safer than traditional narcotic analgesics.

Nalbuphine was found to be effective in the control of severe and deep pain caused by cardiac, pulmonary, abdominal, osteopathia, and obstetrical surgery or severe burn injury and the terminal stages of cancer via the various administration routes, such as intramuscular, intravenous, or intrathecal (Schmit, W.K. et al., 1985). The only limitation of nalbuphine is the short duration of action. Wang, J.J. et al. (Ha. Tsui. Hsueh. Tsa. Chi., vol. 23, 3, 1985) have reported

the effect of nalbuphine can only be sustained for 3-5 hrs after intravenous administration and 6-8 hrs even by intrathecal injection. However, find a new way to state this severe pain usually cannot be relieved in that a short period of tine.

Thereof, any improvement in extending nalbuphine efficacy to a longer acting injection would be a great breakthrough in medicine and at the same time would provide a more economic therapeutic system. The prodrug approach is widely used to increase the duration of drugs which are rapidly eliminated. The antipsychosis agent, haloperidol, is one example, Hemstrom, C.A. et al. (Drug Intell. Clin. Pharm., Vol. 22, 290, 1988) have found that the dosing interval of haloperidol decanoate can be prolonged from 2-4 times a day to 1-2 times a month. Joshi, J.V. et al. (Steroids, Vol. 53, 571, 1989) also reported that the prodrug of northisterone enanthate can be given once every 2 months.

Broekkamp, C.L. et al. (J. Pharm. Pharmacol., Vol.40, 434, 1988) have proposed the long-acting mechanism of action of ester-type prodrugs. They are esterified with fatty acids of different carbon numbers, resulting and increase in the lipophilicity of the prodrugs. Thereof, when prodrugs are given intramuscularly, as the release rates are decreased, the duration of action is prolonged. Ester-type prodrugs are hydrolyzed by esterases in the body resulting in the increase of mother compounds. Esterase exists in many tissues and organs, such as blood, brain, liver, heart, lungs, kidneys, and muscles. The pharmacological effect and safety of the ester-type prodrug and mother compound are reported to be the same (Gelders, Y.G. et al., Int. Clin. Psychopharmacol., Vol. 1, 1, 1986).

EP-0170090-A discloses substituted benzoate ester prodrug derivatives of 3-hydroxymorphinans which are said to provide enhanced bioavailability of 3-hydroxymorphinans from orally administered doses.

DE-2323192-A discloses injectable long-acting narcotic antagonist compositions comprising naloxone or derivatives thereof and a vegetable oil.

Morphine is a narcotic analgesic with an addiction effect. Its structure was shown in Figure (3). Broekkamp, C.L. et. al (1988) have stated that esterification of the phenol group at the 3rd position of the morphinan ring makes esterifical derivatives which have the following characteristics. (1) increased lipophilicity, (2) low affinity to morphine receptors, as side effects are decreased, but the released parent drugs have the same pharmacological activity, (3) the effect and safety of esterifical derivatives and mother compounds are the same.

Figure (3)

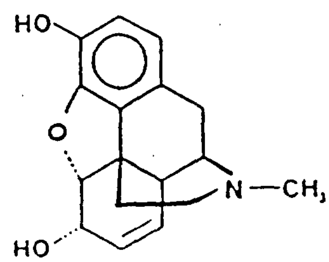

MORPHINE

It is also possible to decreased the release rate and side effects of a narcotic analgesic using subcutaneous administration. The subcutaneous area part has less blood flow and more fatty tissue than other areas of the body, the ester-type prodrugs are slowly hydrolyzed by esterases in the subcutaneous area. Using the intracerebroventricular or spinal cord administration,Rutter, D. V. et al. (Br. J. Anesth., vol. 53, 915,1981) also studied the increase in the duration of action and the decrease in side effects of narcotic analgesics such as Morphine, Fentanyl, Buprenorphine, Nalbuphine. Other than those administration methods described above, the percutaneous method is also favorable. Hill, H.F. et al.(Eur.J. Pharmacol., vol.119, 23, 1985) have proposed the Percutaneous administration of Fentanyl. But the Fentanyl percutaneous dosage form of safety range is 0.6-3 μ g/ml the same as the narcotic analgesics of the Mu receptor. Although Bruce et al. in U.S. Pat. No. 4,673,6779 issued in June. 16,1987 disclosed specifically that Morphine, Fentanyl, Buprenorphine, Nalbuphine derivatives enhanced bioavailability from sublingual, buccal, nasal dosage forms, but it was unconcerned with the long acting effect of Nalbuphine prodrugs and the specific dosage forms as this invention has described.

Nalbuphine prodrugs are relatively new analgesics as shown in Figure 1, while R group is the R'CO, the R' is selected from the groups consisting of (a) straight chain alkyl group, (b) branched chain alkyl group, (c) straight chain alkyl group on benzene (d) branched chain alkyl group on benzene, wherein the straight chain branched chain is represents the saturated or unsaturated C1-40 alkyl groups. The straight chain branched chain alkyl group on benzene is represents the saturated or unsaturated C6-40 alkyl groups. There not only possess the potent analgesic activity of the commonly used analgesics, but also minimize side effects, such as addiction and respiratory depression associated with traditional narcotics. In order to develop approaches for prolonging the clinical efficacy of nalbuphine, this present

invention relates formulations capable of prolonging the release of nalbuphine and the design of nalbuphine prodrugs by modifying the chemical structure of nalbuphine. The nalbuphine prodrugs are characterized by (1) long-acting, i.e. when given parenterally, ( for example: intramuscularly) the dosing interval can be altered from every four hours to several days, or even longer, (2) low first-pass effect and high bioavailability when given orally.

Figure (1)

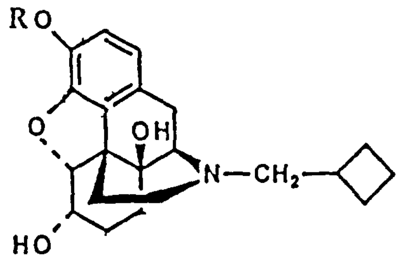

This Present invention also comprises the preparations of ester-type nalbuphine prodrugs as shown in Figure 1. Nalbuphine esters were synthesized by the following steps: (1) chlorinate the hydroxyl group of nalbuphine base by reacting with methylene chloride, (2) react with triethylamine which was dissolved in methylene chloride, (3) proceed the esterification by reacting with various lengths of fatty acid anhydrides or acid chlorides, (4) purify the above product by silica chromatography. Otherwise, the nalbuphine esters can be obtained by the general methods of preparing esters from alcohols or phenols, for instance, by reacting the hydroxyl group with acid chlorides, acid anhydrides, carboxylic acids, esters, or sulfonyl chlorides.

The preferred reactants for preparing nalbuphine prodrugs are propionic acid, benzoic acid, enanthic acid, n-valeric acid, pivalic acid, decanoic acid and saturated fatty acids, such as lauric acid, stearic acid, arachidic acid, cerotic acid, etc. and unsaturated fatty acid, such as oleic acid, linolenic acid, undecylenic acid, cinnamic acid, etc. In Figure 1, R is the fatty acid anhydride which containg various lengths of fatty acid anhydrides, and is denoted by R'CO. In which R' is selected from the groups consisting of (a) straight chain alkyl groups, (b) branched-chain alkyl groups, (c) straight chain alkyl groups on benzene (d) branched chain alkyl groups on benzene , wherein the straight chain branched chain is represents the saturated or unsaturated C1-40 alkyl groups. The straight chain branched chain alkyl group on benzene is represents the saturated or unsaturated C6-40 alkyl groups.

The nalbuphine prodrugs synthesized by the above-mentioned methods were identified by nuclear magnetic resonance (NMR), infrared (IR) and ultraviolet (UV) spectroscopy, gas chromatography/mass spectrometry (GC/MS) and elemental analysis and then formulated into the different dosage forms as desired. Suitable dosage forms of nalbuphine prodrugs can be oral preparations, oily injectable with sesame oil, soybean oil, or peanut oil as vehicles for injection. The oily injectable can be given by intracerebroventricular, intramuscular, subscutaneous, or spinal marrow etc. routes. The nalbuphine prodrugs can be formulated and administered by percutaneous, buccal, sublingular, topical (e.g. pastes, ointments, suppositories) routes as well.

In this invention, a novel animal model for testing the antinociceptive action of analgesics was also established. Traditionally, analgesics are evaluated by infrared tail-flick, hot water tail-flick, hot plate tests or writhing tests. However, these animal models proved to be inapplicable for the narcotic agonist-antagonists, such as buprenorphine, nalbuphine and butorphanol (Shaw, J.S., Br. J. Pharmacol. VOl. 195, 578, 1988). Besides, animals usually died after performing the writhing test. Thereof, the writhing test is not suitable for long-acting analgesics. In the present animal model, nociceptive thresholds were measured before and after drug dosing as the latency for the Sprague-Dawley rat to flick or remove its tail from the cold ethanol (-20° C) bath after immersion. This latency was used as an indicator for the duration of action of the tested analgesic. This animal model is the so-called rat cold ethanol tail-flick test.

Example 1 Preparation of nalbuphine propionate

75 mL of methylene chloride and 3.75 g ( 0.01 mole) of nalbuphine were added to a 250-mL round-bottomed flask. The flask was placed in an ice bath to keep cool. The contents was stirred and then gradually added to a 20 mL methylene chloride solution containing 0.16 mole of triethylamine. With rapid stirring, another 20 mL of methylene chloride solution containing 0.011 mole of dry propionic anhydride was added drop by drop. Afterwards, the mixture was continuously stirred under room temperature for 1 hour. 20 mL of 10% sodium carbonate solution was added to neutralize the residual acids and remove the water soluble impurities. Sodium sulfate was used to dehydrate the solution. After drying under vacuum, a nalbuphine propionate solid was obtained. The product was purified by column chromatography. Physical data are shown in Tables 1 and and Figures 4 and 5.

Tab. 1

| | R | MW | MF | MP |
|---|---|---|---|---|
| Nalbuphine | H | 357.46 | $C_{21}H_{27}NO_4$ | 222-223 °C |
| N-Decanoate | 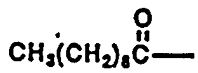 | 511.70 | $C_{31}H_{45}NO_5$ | 75-76 °C |
| N-Enanthate | 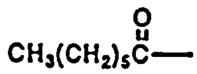 | 469.62 | $C_{28}H_{39}NO_5$ | 75-77 °C |
| N-Benzoate | 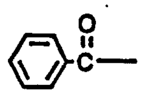 | 461.56 | $C_{28}H_{31}NO_5$ | 153-156 °C |
| N-Pivalate | 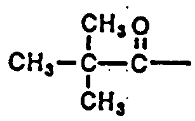 | 441.59 | $C_{26}H_{36}NO_5$ | 91-93 °C |
| N-Propionate | 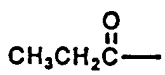 | 413.51 | $C_{24}H_{31}NO_5$ | 144-145 °C |

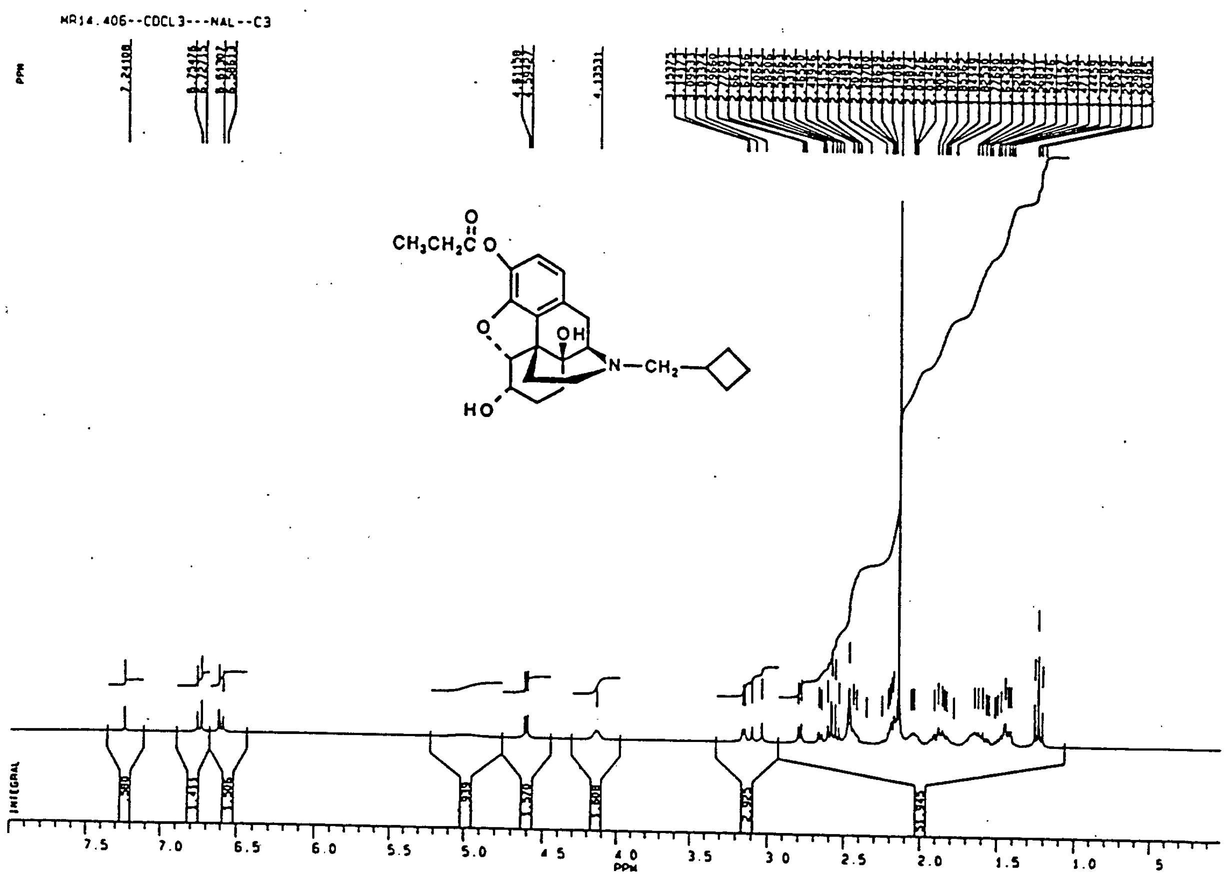


Fig. 4

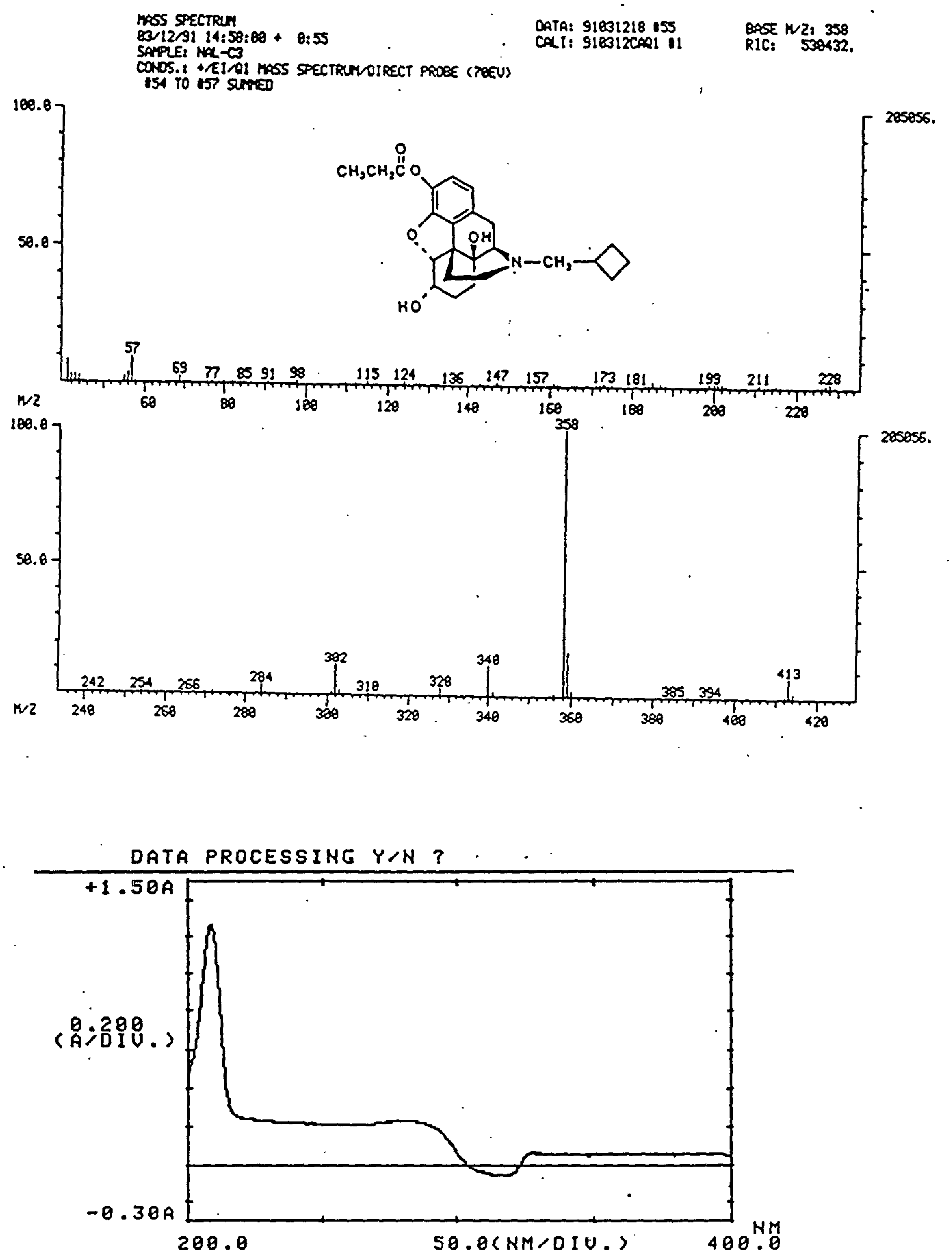
MASS SPECTRUM
03/12/91 14:58:00 + 0:55
SAMPLE: NAL-C3
CONDS.: +/EI/Q1 MASS SPECTRUM/DIRECT PROBE (70EV)
#54 TO #57 SUMMED
DATA: 91031218 #55
CALI: 910312CAQ1 #1
BASE M/Z: 358
RIC: 530432.
100.0
50.0
205056.
M/Z
DATA PROCESSING Y/N ?
+1.50A
0.200 (A/DIV.)
-0.30A
200.0
50.0(NM/DIV.)
400.0
NM

Fig. 5

Example 2 Preparation of nalbuphine pivalate,

75 mL of methylene chloride and 3.75 g ( 0.01 mole) of nalbuphine were placed in a 250-mL ice-bathed, round-bottomed flask. While stirring, 0.01 mole of pivaloyl chloride (dissolved in 20 mL of methylene chloride) vas gradually added. Following the procedure as described in Example 1, a pure nalbuphine pivalate was obtained. Physical data are shown in Table 1 and Figures 6, 7 and 8.

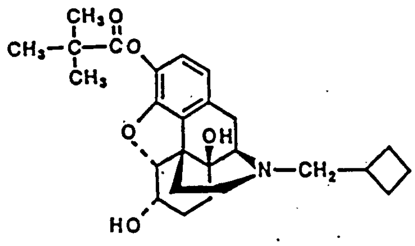

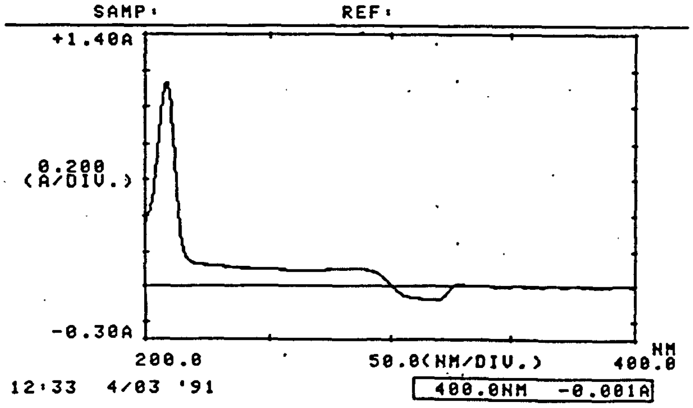


| λ | ABS | λ | ABS |
|---|---|---|---|
| 400.0 | -0.004 | 395.0 | -0.002 |
| 390.0 | -0.003 | 385.0 | -0.003 |
| 380.0 | -0.004 | 375.0 | -0.003 |
| 370.0 | -0.003 | 365.0 | -0.003 |
| 360.0 | -0.004 | 355.0 | -0.003 |
| 350.0 | -0.004 | 345.0 | -0.004 |
| 340.0 | -0.002 | 335.0 | -0.000 |
| 330.0 | 0.006 | 325.0 | -0.008 |
| 320.0 | -0.073 | 315.0 | -0.077 |
| 310.0 | -0.069 | 305.0 | -0.053 |
| 300.0 | 0.003 | 295.0 | 0.065 |
| 290.0 | 0.094 | 285.0 | 0.099 |
| 280.0 | 0.096 | 275.0 | 0.094 |
| 270.0 | 0.088 | 265.0 | 0.089 |
| 260.0 | 0.089 | 255.0 | 0.094 |
| 250.0 | 0.095 | 245.0 | 0.097 |
| 240.0 | 0.103 | 235.0 | 0.107 |
| 230.0 | 0.114 | 225.0 | 0.123 |
| 220.0 | 0.130 | 215.0 | 0.225 |
| 210.0 | 1.046 | 205.0 | 0.761 |
| 200.0 | 0.351 | | |

Fig. 6

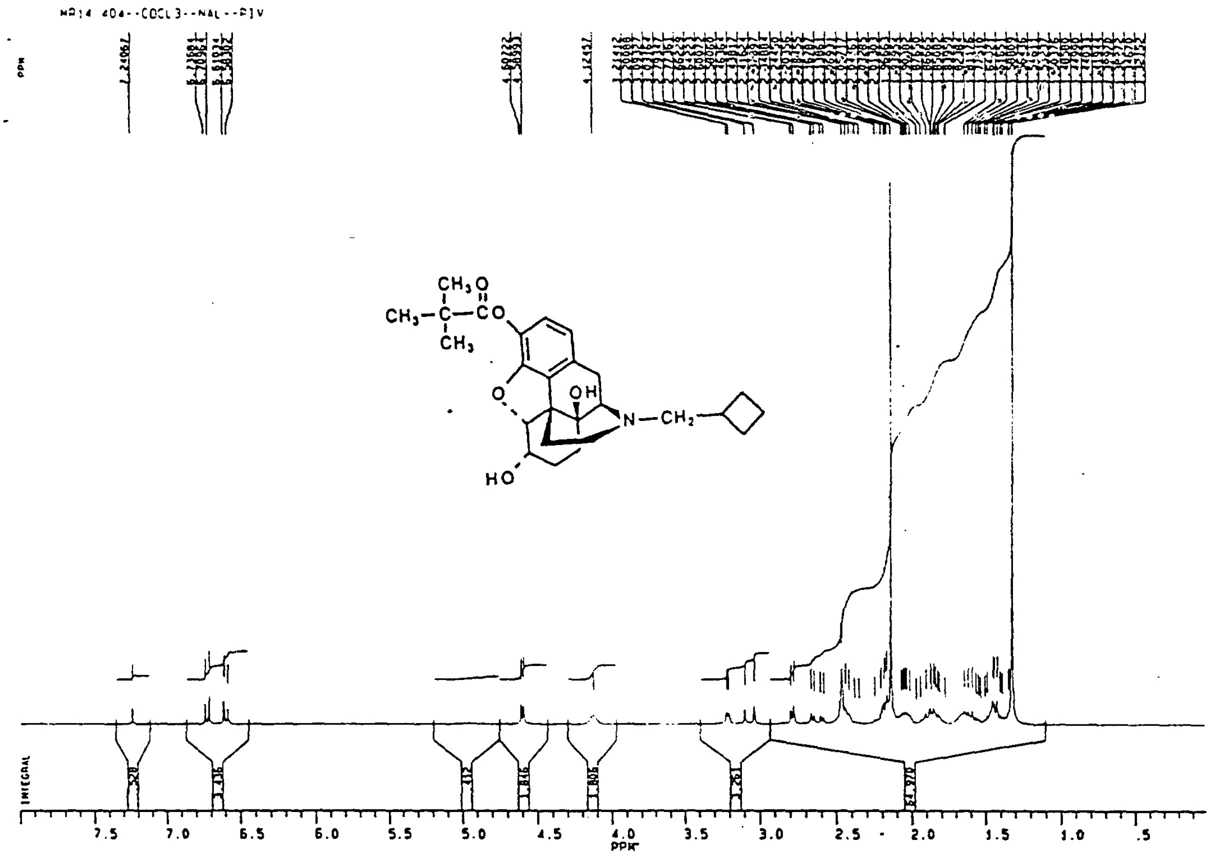

Fig. 7

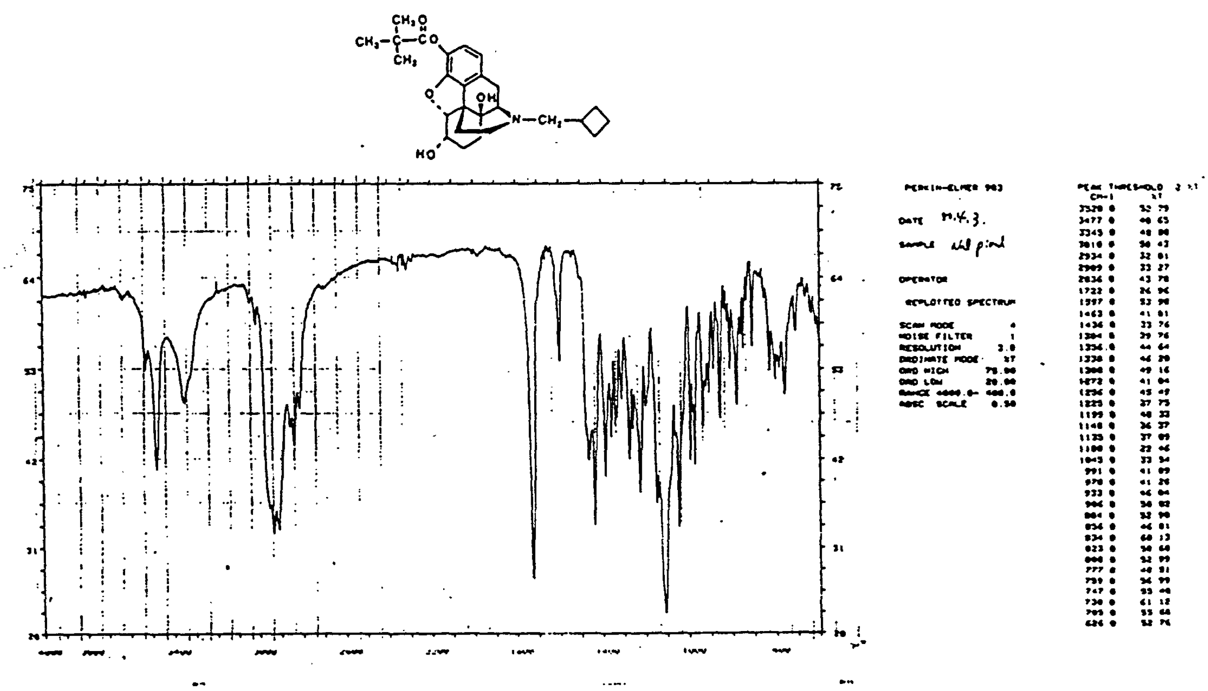

Fig. 8

Example 3 Preparation of nalbuphine benzoate

According to the procedure of Example 1, wherein 0.011 mole of benzoyl chloride was substituted for anhydrous propionic acid. Thus, a pure nalbuphine benzoate was obtained. Physical data are shown in Table 1.

Example 4 Preparation of nalbuphine enanthate

According to the procedure of Example 1, wherein 0.011 mole of heptanoyl chloride was substituted for anhydrous propionic acid. Thus, a pure nalbuphine enanthate was obtained. Physical data are shown in Table 1 and Figure 9.

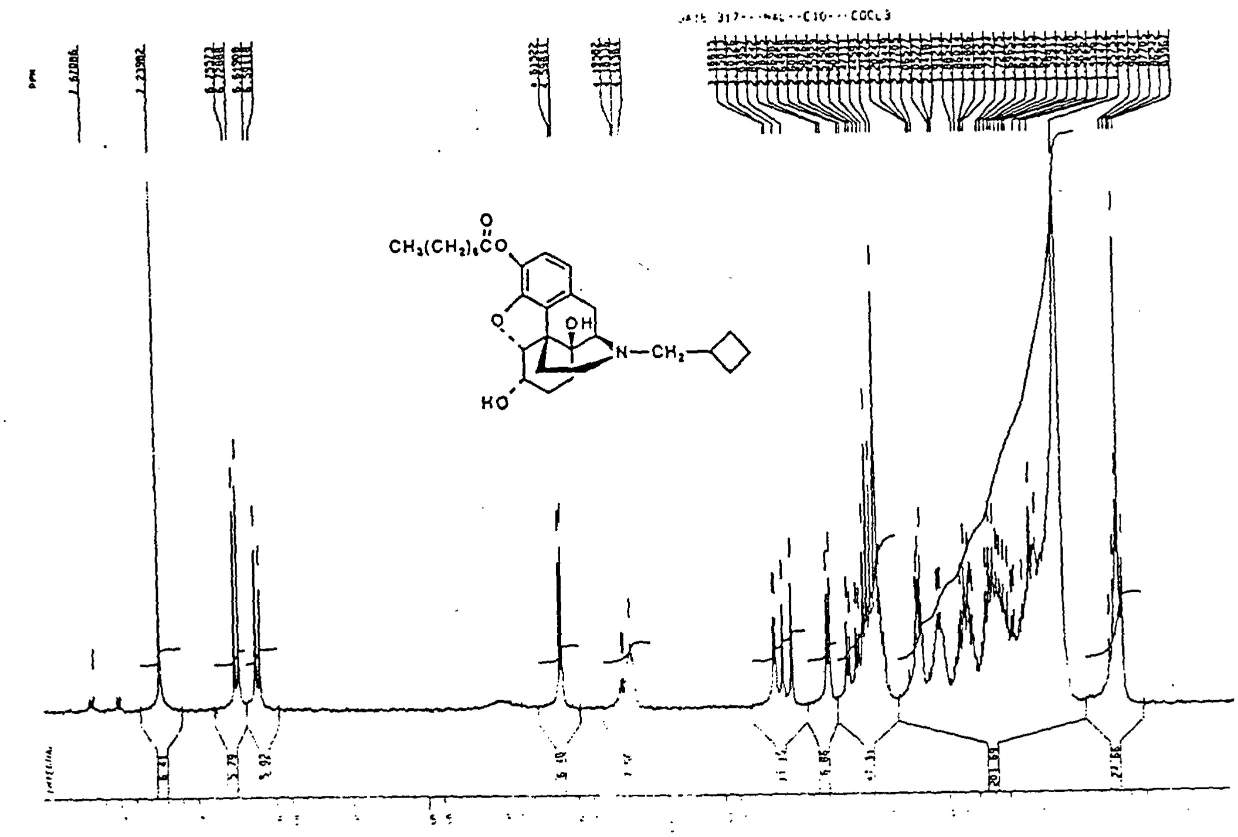

Fig. 9

Example 5 Preparation of nalbuphine decanoate

According to the procedure of Example 1, wherein 0.011 mole of decanoyl chloride was substituted for anhydrous propionic acid. Thus, a pure nalbuphine decanoate was obtained. Physical data are shown in Table 1 and Figure 10.

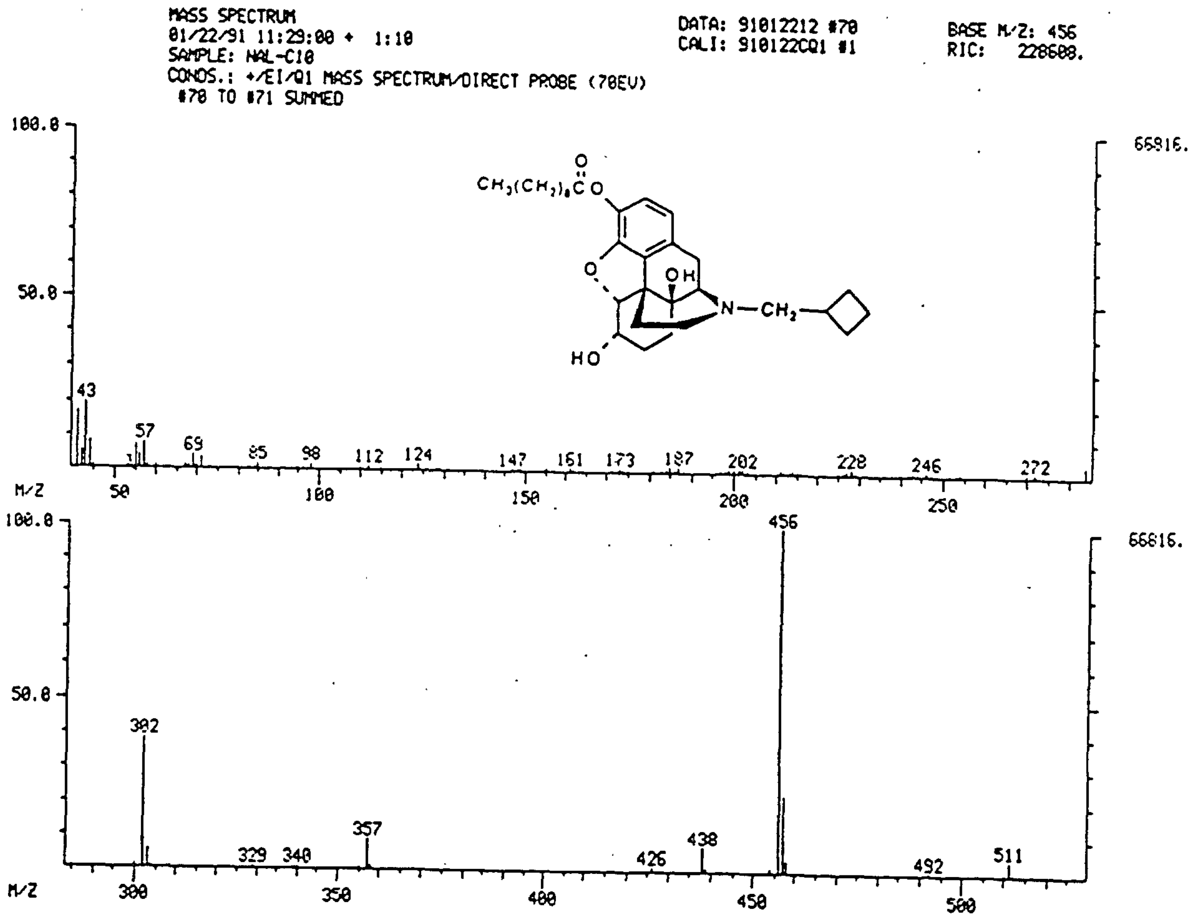


Fig. 10

Example 6 Preparation of nalbuphine behenate

According to the procedure of Example 1, wherein 0.35 g of behenic anhydride was substituted for anhydrous propionic acid. Thus, a pure nalbuphine behenate was obtained. Physical data are shown in Figure 11.

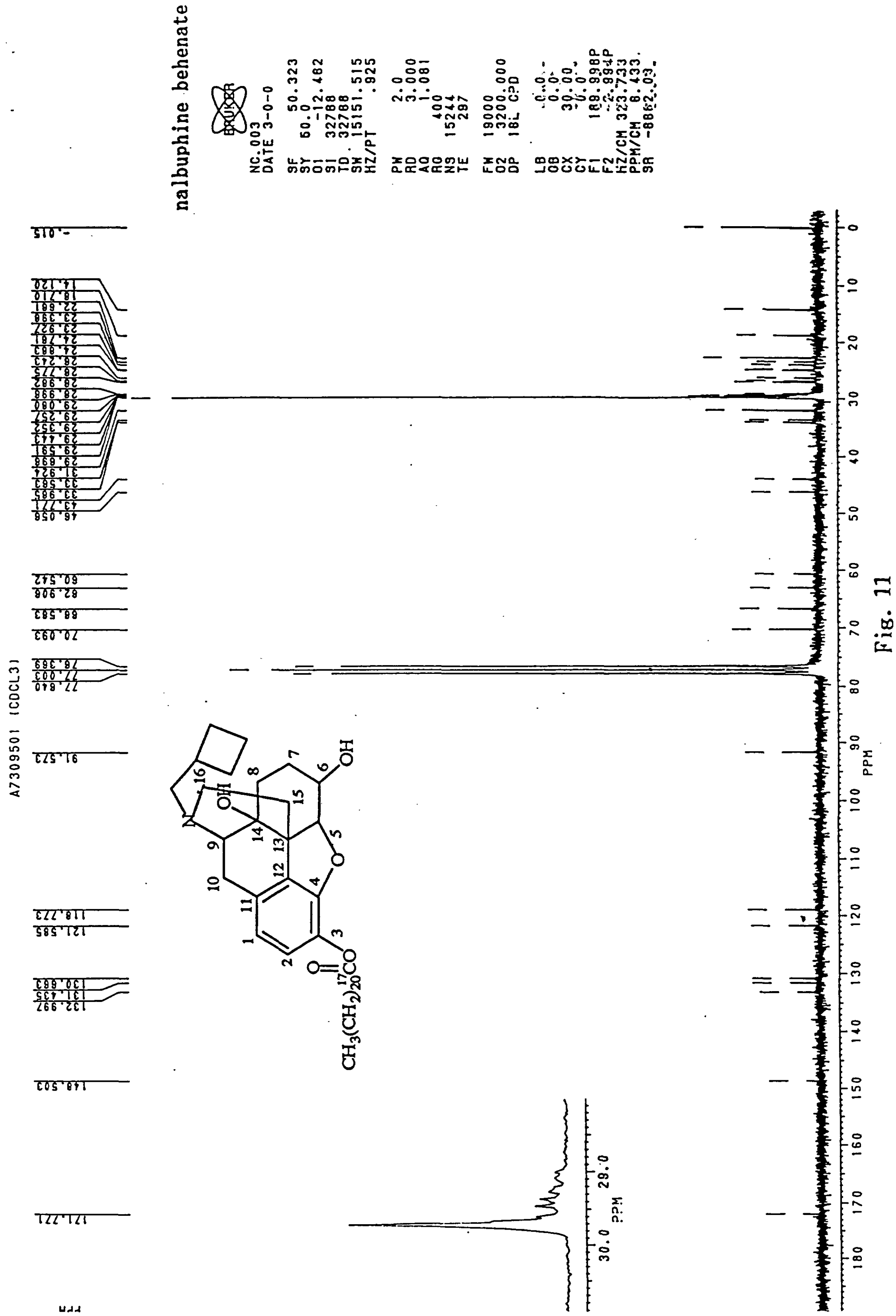
nalbuphine behenate
NC.003
DATE 3-0-0
SF 50.323
SY 60.0
O1 -12.482
SI 32768
TD 32768
SW 15151.515
HZ/PT .925
PW 2.0
RD 3.000
AQ 1.081
RG 400
NS 15244
TE 297
FW 19000
O2 3200.000
DP 18L CPD
A7309501 (CDCL3)
PPM

Fig. 11

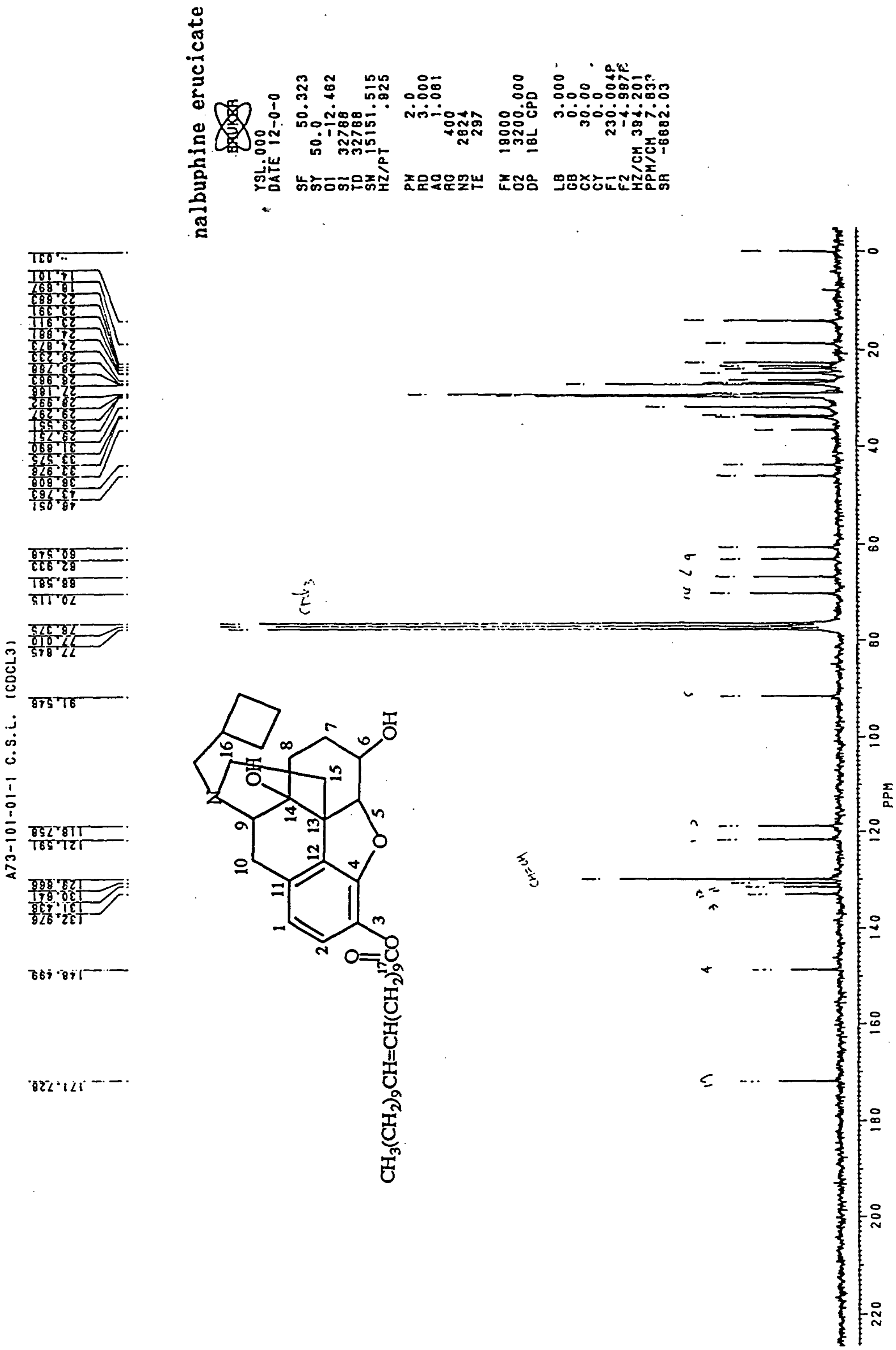
nalbuphine erucicate
BRUKER
YSL.000
DATE 12-0-0
SF 50.323
SY 50.0
O1 -12.482
SI 32768
TD 32768
SW 15151.515
HZ/PT .925
PW 2.0
RD 3.000
AQ 1.081
RG 400
NS 2824
TE 297
FW 19000
O2 3200.000
DP 16L CPD
LB 3.000
GB 0.0
CX 30.00
CY 0.0
F1 230.004P
F2 -4.997P
HZ/CM 384.201
PPM/CM 7.633
SR -6882.03
A73-101-01-1 C.S.L. (CDCL3)
14.101
18.697
22.683
23.391
23.911
24.881
26.233
26.788
26.963
27.166
28.992
29.297
29.551
29.751
31.890
33.575
33.978
38.608
43.763
48.051
60.548
62.333
66.581
70.115
76.375
77.010
77.645
91.548
118.758
121.591
129.866
130.841
131.438
132.978
148.499
171.728
PPM

Fig. 12

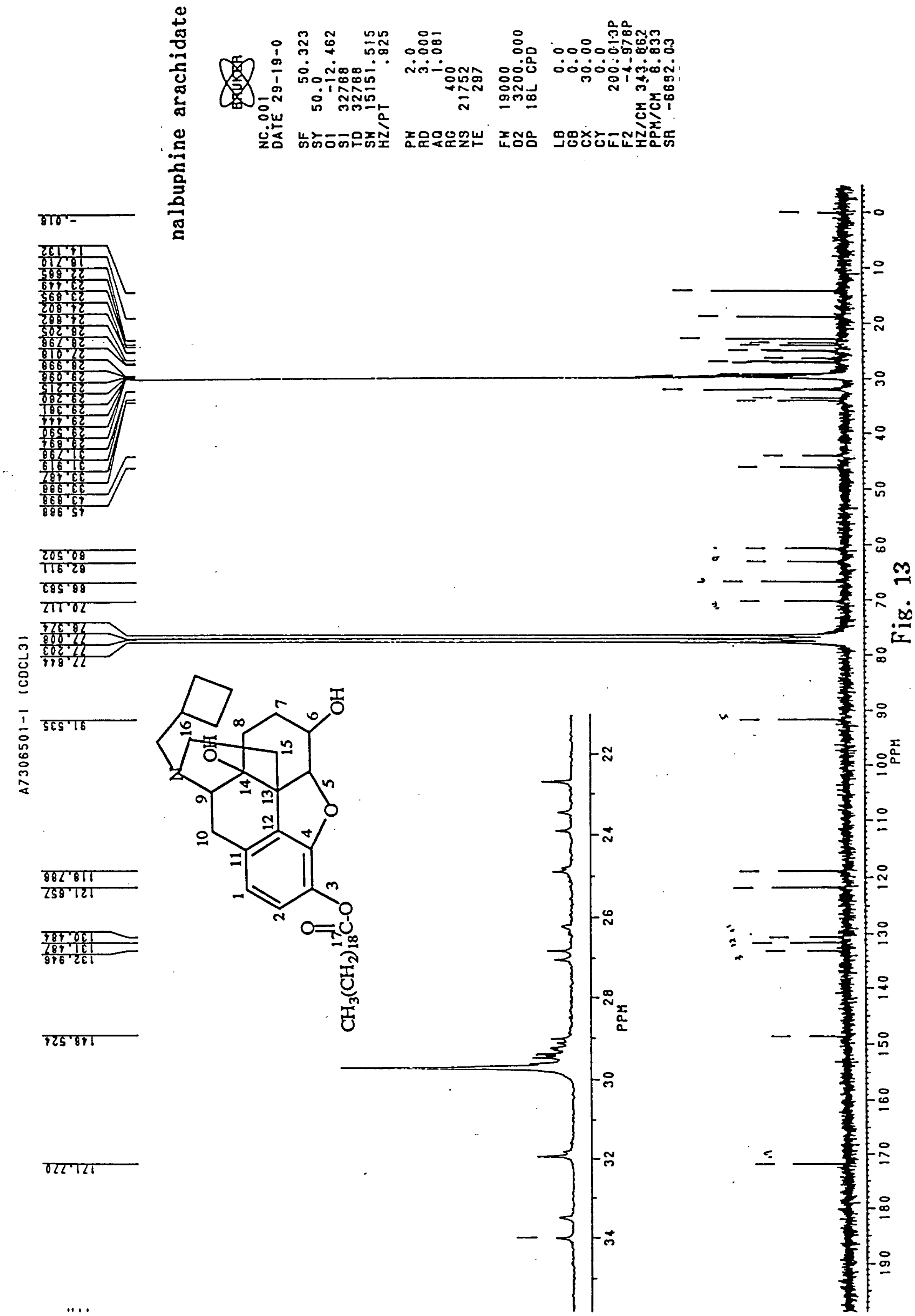

Fig. 13

Example 7 Preparation of nalbuphine erucicate.

According to the procedure of Example 1, wherein 0.48 g of erucic anhydride was substituted for anhydrous propionic acid. Thus, a pure nalbuphine erucicate was obtained. Physical data are shown in Figure 12.

Example 8 Preparation of nalbuphine arachidate

According to the procedure of Example 1, wherein 0.85 g of arachidic anhydride was substituted for anhydrous propionic acid. Thus, a pure nalbuphine arachidate was obtained. Physical data are shown in Figure 13.

Example 9 The selection of oil for injection of nalbuphine prodrugs

Materials

Sesame oil, soybean oil, and an ethyl ester of peanut oil were tested in the present invention as oil vehicles for nalbuphine prodrugs. Phosphate buffer (monobasic potassium phosphate 1.9 g, dibasic sodium phosphate 8.1 g, and sodium chloride 4.11 g were dissolved in 1 L water to make an isotonic solution of pH 7.4) was used as a control.

Experimental

Each experimental group had six samples. Placed into a dialysis bag were 50 mg ( 0.127 mmole base ) of nalbuphine hydrochloride or 45 mg (0.127 mmole) of nalbuphine free base and 1 mL of the oil vehicle or the phosphate buffer cut off 12,000-14,000). A 250-mL of iodine flask containing 150 mL of phosphate buffer was used to commodity the dialysis bag. Inside the flask, was placed a magnetic stirrer bar. The dialysis Proceed at a stirring speed of 500 rpm and measured the release rate of nalbuphine from each preparation. A UV spectrometer (UV-160, Shimadzua, Kyoto, Japan ) was used to detect the nalbuphine content in the phosphate buffer outside the bag.

Result:

The release profile of nalbuphine from each preparation and control group are shown in Figures 14, 15 and 16 As shown in Figure 14, the preparation with sesame oil as the vehicle has the slowest release rate ($p<0.05$) and there was no significant difference within the other three preparations. Figure 15 shows the amount of released nalbuphine free base was less in preparations with sesame oil than with peanut oil ester and the control between 17 and 28 hours ($p<0.05$). In Figure 16, the released amount of nalbuphine from preparation (A) nalbuphine hydrochloride dissolved in sesame oil, preparation (B) nalbuphine hydrochloride dissolved in phosphate buffer, and preparation (C) nalbuphine free base dissolved in sesame oil were compared with one another. During the first three 3 hours, (B) was greater than (A), and (A) greater than (C). During the time between 3 and 28 hours, (A) was greater than (C).

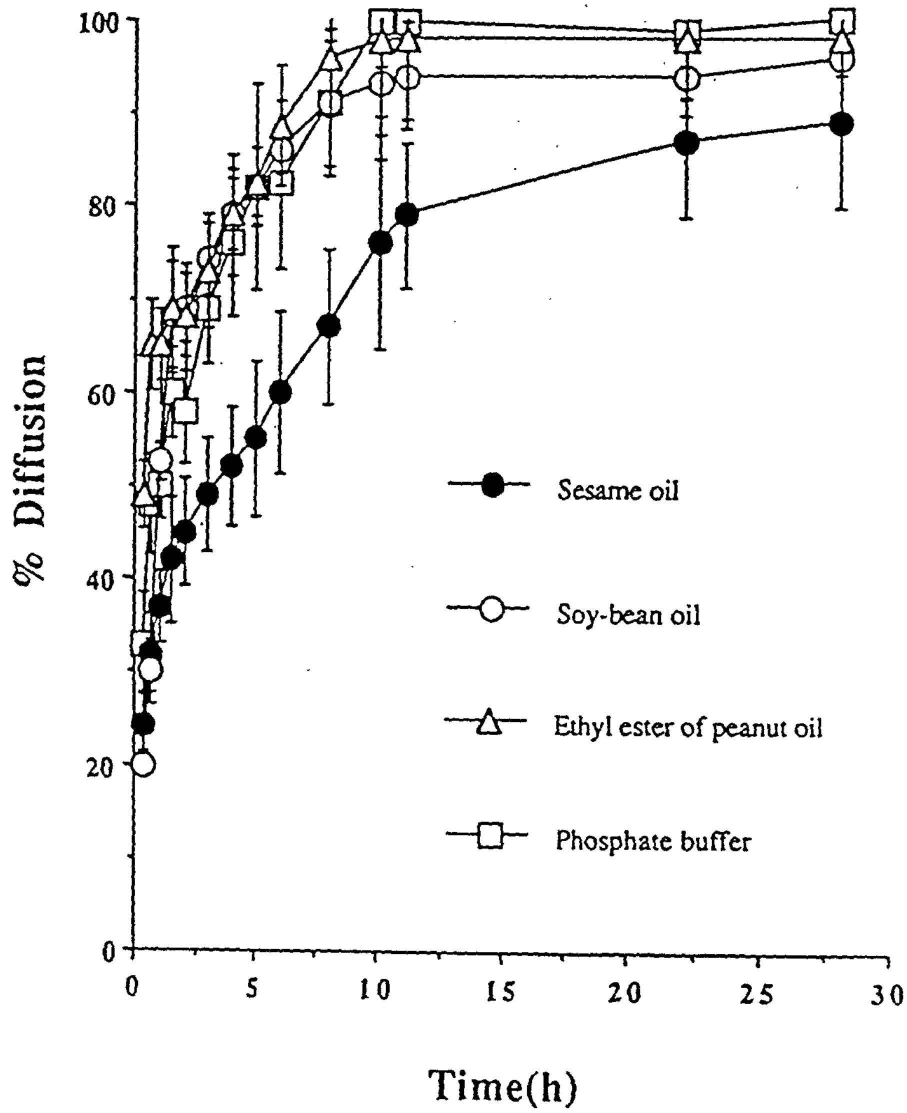
100
80
60
40
20
0
% Diffusion
0
5
10
15
20
25
30
Sesame oil
Soy-bean oil
Ethyl ester of peanut oil
Phosphate buffer
Time(h)

Fig. 14

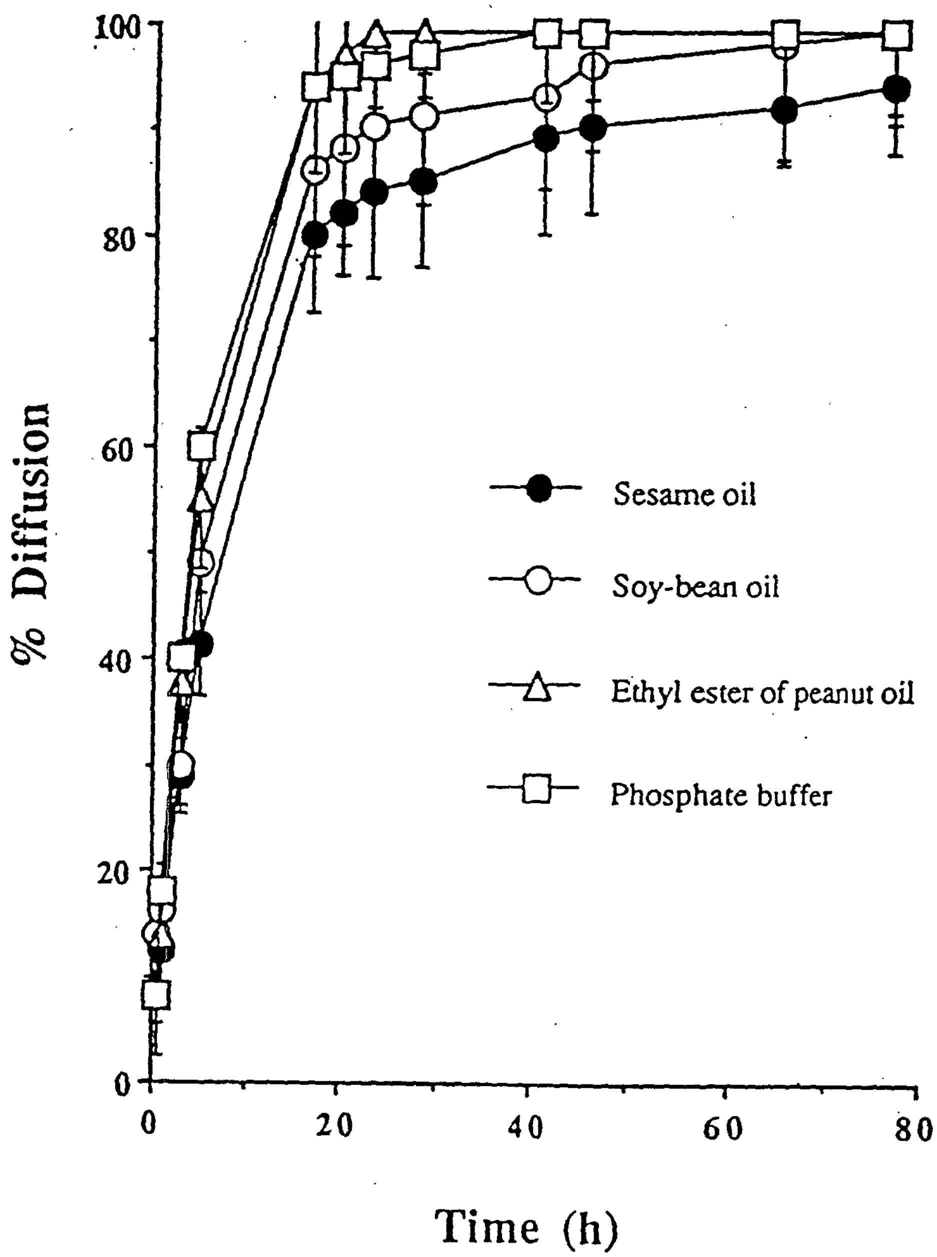
100
80
60
40
20
0
% Diffusion
0
20
40
60
80
Time (h)
Sesame oil
Soy-bean oil
Ethyl ester of peanut oil
Phosphate buffer

Fig. 15

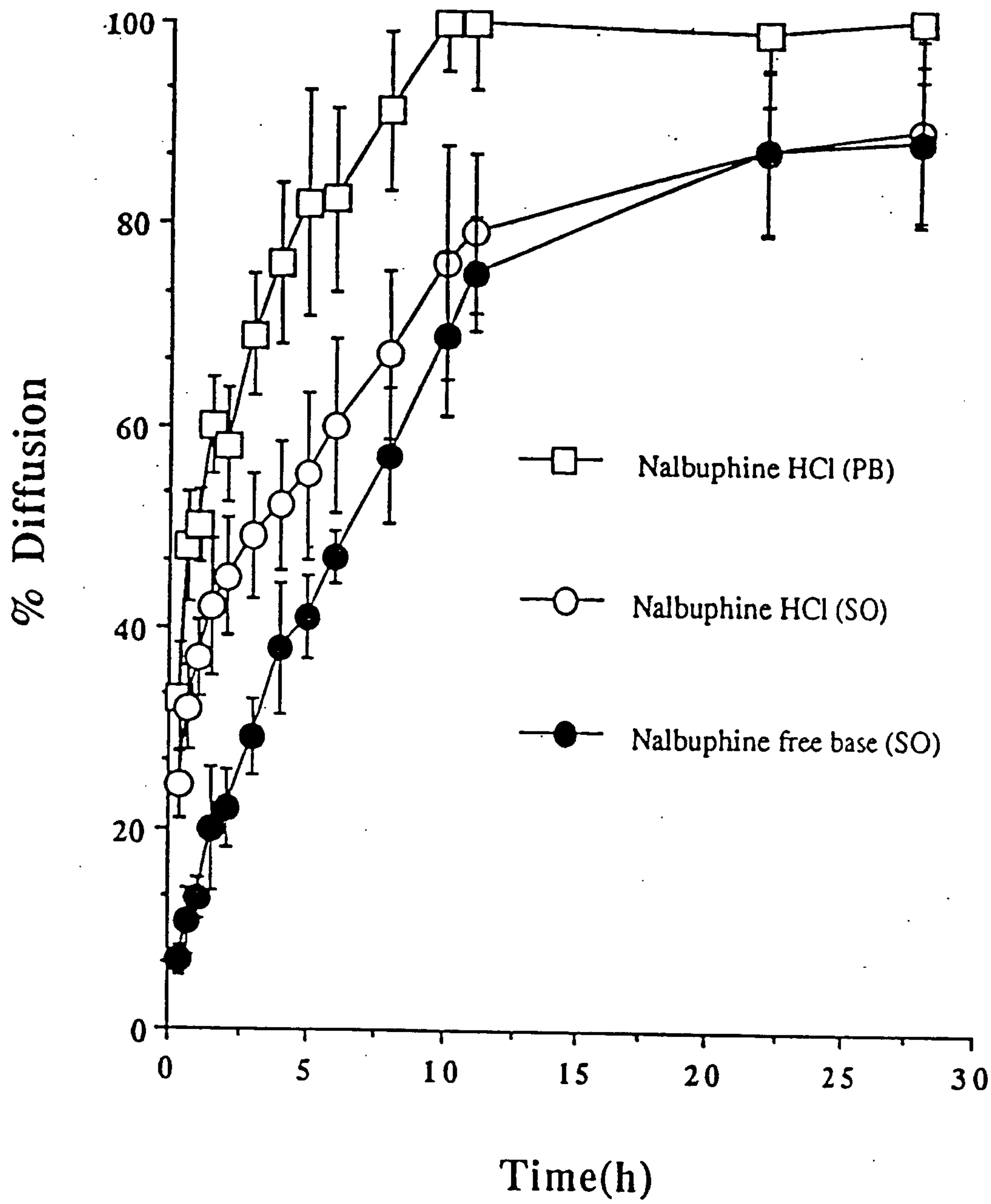


Fig. 16

Example 10 preparation of long-acting nalbuphine prodrugs oily injections

(1) 10.50 mg of nalbuphine propionate was added to 2.8 mL of sesame oil and shaken slightly to effectively complete dissolution.
(2) 11.91 mg of nalbuphine enanthate was then added to 2.8 mL of sesame oil and shake slightly to effectively complete dissolution.

Example 11 The preparation of long-acting nalbuphine prodrug saturated oily injectable suspension

300 mg of nalbuphine enanthate was added to 2.8 mL of sesame oil (supersaturated), resulting in a saturated oily injectable suspension.

Example 12 The preparation of oral capsules

To a mixture of 4.1 g of nalbuphine propionate and 10 mL of tetrahydrofuran, 2 mL of 20% hydrochloric acid alcoholic solution was added drop by drop. A monohydrochloride salt of nalbuphine propionate was precipitated. About 4.4 g of water soluble nalbuphine propionate monohydrochloride was thus obtained. The product was placed in hard gelatin capsules or other capsules for pharmaceutical use.

Example 13 The preparation of percutaneous dosage form

470 mg of nalbuphine enanthate was added to an aqueous solution containing 50% glycerin and 50% of 20 ng/mL methylcellulose to make a gel preparation by triturated.

Example 14 *In vivo* pharmacodynamic study prodrugs

(1) Animal

Male, Sprague-Dawley rats (175-225 g) were used. Each group consisted of 6 rats, each injected once intramuscularly in the rear leg.

(2) material

A. The Dose-Response Curves

(a) Nalbuphine hydrochloride doses of 100 mg/kg, 10 mg /kg, 0.5mg/kg, 0.1 mg/kg, 0.05 mg/kg, 0.01 mg/kg were used.
(b) Morphine hydrochloride doses of 10 mg/kg, 5 mg/kg, 1 mg/kg, 0.5 mg/kg, 0.1 mg/kg, 0.05 mg/kg, 0.01 mg/kg were used.
(c) Buprenorphine hydrochloride doses of 100 mg/kg, 10 mg/kg ,5 mg/kg, 1 mg/kg, 0.5 mg/kg, 0.1 mg/kg, 0,01 mg/kg were used.

B. The Pharmacodynamic Study of Nalbuphine Prodrugs

(a) 25 micromole/2.8 ml of nalbuphine hydrochloride in saline and nalbuphine base in sesame oil were used as controls. The dose for each rat was 25 micromole per kg, intramuscularly.
(b) 25 micromole/2.8 ml of Nalbuphine prodrugs in sesame oil were used for study. The dose for each rat was 25 micromole per kg, intramuscularly.

(3) Experimental

In this experiment, a circulating cold ethanol bath with a temperature maintained at -20 C was set-up. After dosing, the rat tail (1/3 from the tip) was immersed in the bath. The latency for the rat to flick its tail from the bath was measured to be the nocioceptive threshold. The effect of various opium alkaloid preparations can be applied to this test.
The nocioceptive effect can be calculated as follows:

$$\text{The percentage of nocioceptive effect} = \frac{\begin{bmatrix}\text{Latency}\\ \text{after dosing}\end{bmatrix} - \begin{bmatrix}\text{Latency}\\ \text{before dosing}\end{bmatrix}}{\begin{bmatrix}\text{experimental}\\ \text{end}\end{bmatrix} - \begin{bmatrix}\text{Latency}\\ \text{before dosing}\end{bmatrix}} \times 100$$

35, 25 and 15 minutes before dosing, male Sprague Dawley rats were tested to measure basic response latency. The time to stop experiment was set at 40 sec. to prevent the tail from developing a cold sore. No cold sores were found at 40 sec. Five minutes after the drug was administrated to the rat, the flick test was performed not less then every 10 minutes, and in some instances even more.

(4) Results

A. The Dose-Response Curves

The maximal analgesic effect for intramuscular injection was found at 0.05 to 10 mg/kg for morphine hydrochloride, 0.05 to 100 mg/kg for nalbuphine hydrochloride, and 0.1 to 100 mg/kg for buprenorphine hydrochloride (as shown in Fig. 17) .

B. The Pharmacodynamic Study of Nalbuphine Prodrugs

The lipophilicity of nalbuphine base is enhanced when it is prepared from nalbuphine hydrochloride. In Figure 18 the release times are significantly increased. The 50 % analgesic effect for human intramuscular injection was found at 0.8 mmole/kg is 2.5 hours (for rats 25 mmole/kg is 1.1 hours). The 20% analgesic effect for humans is 4.4 hours (for rats 2.1 hours). The 10% analgesic effect for humans is 5.0 hours (rats 2.4 hours). As shown in table 2, as the 50% analgesic effect of nalbuphine hydrochloride it is 2.5 hours, and for nalbuphine propionate 10 hours, with the longest duration of 6.7 days with nalbuphine decanoate. As the 10% analgesic effect of nalbuphine hydrochloride is 12 hours, the longest duration is at 8.2 days of nalbuphine decanoate treatment.

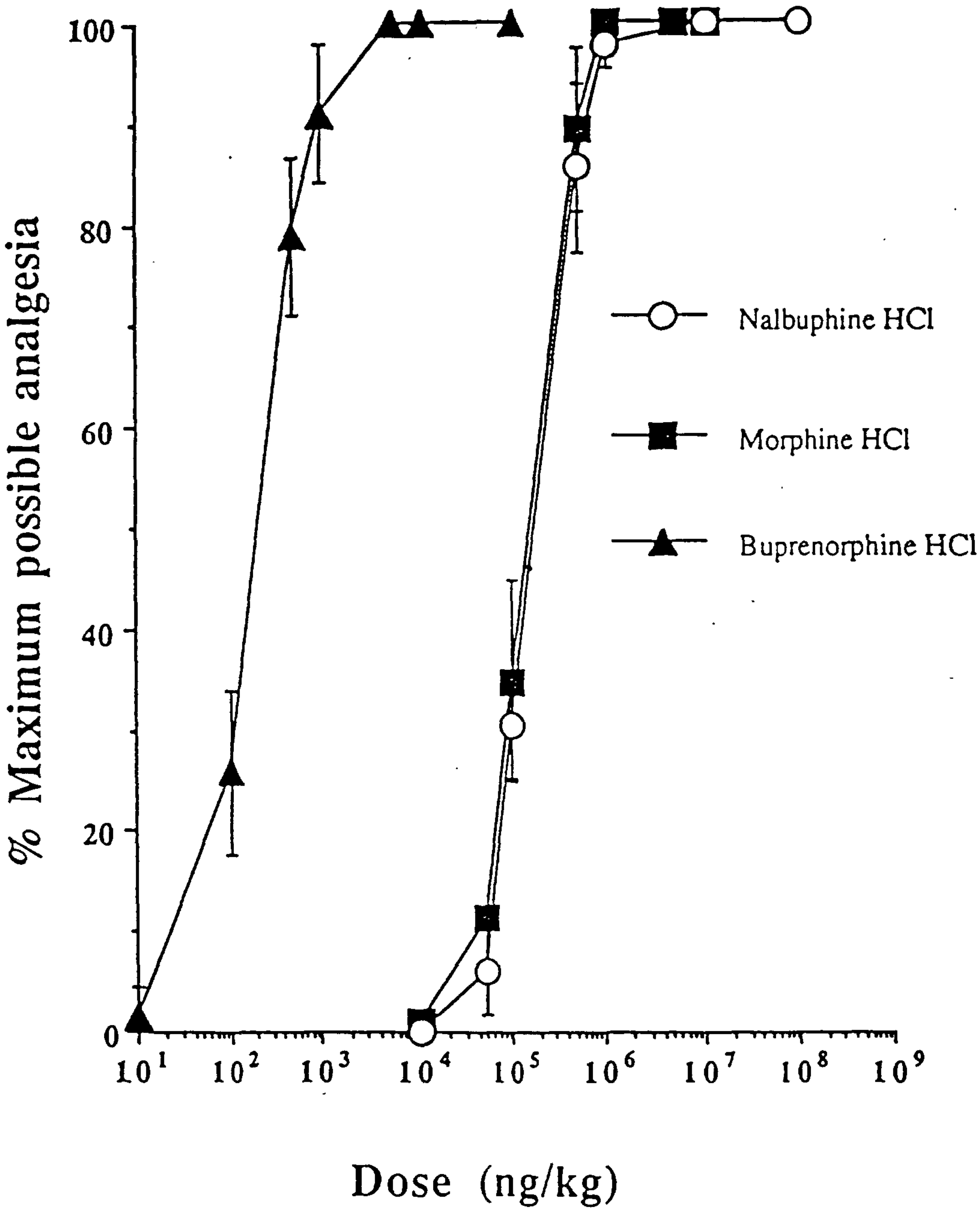
100
80
60
40
20
0
% Maximum possible analgesia
Nalbuphine HCl
Morphine HCl
Buprenorphine HCl
10 1
10 2
10 3
10 4
10 5
10 6
10 7
10 8
10 9
Dose (ng/kg)

Fig. 17

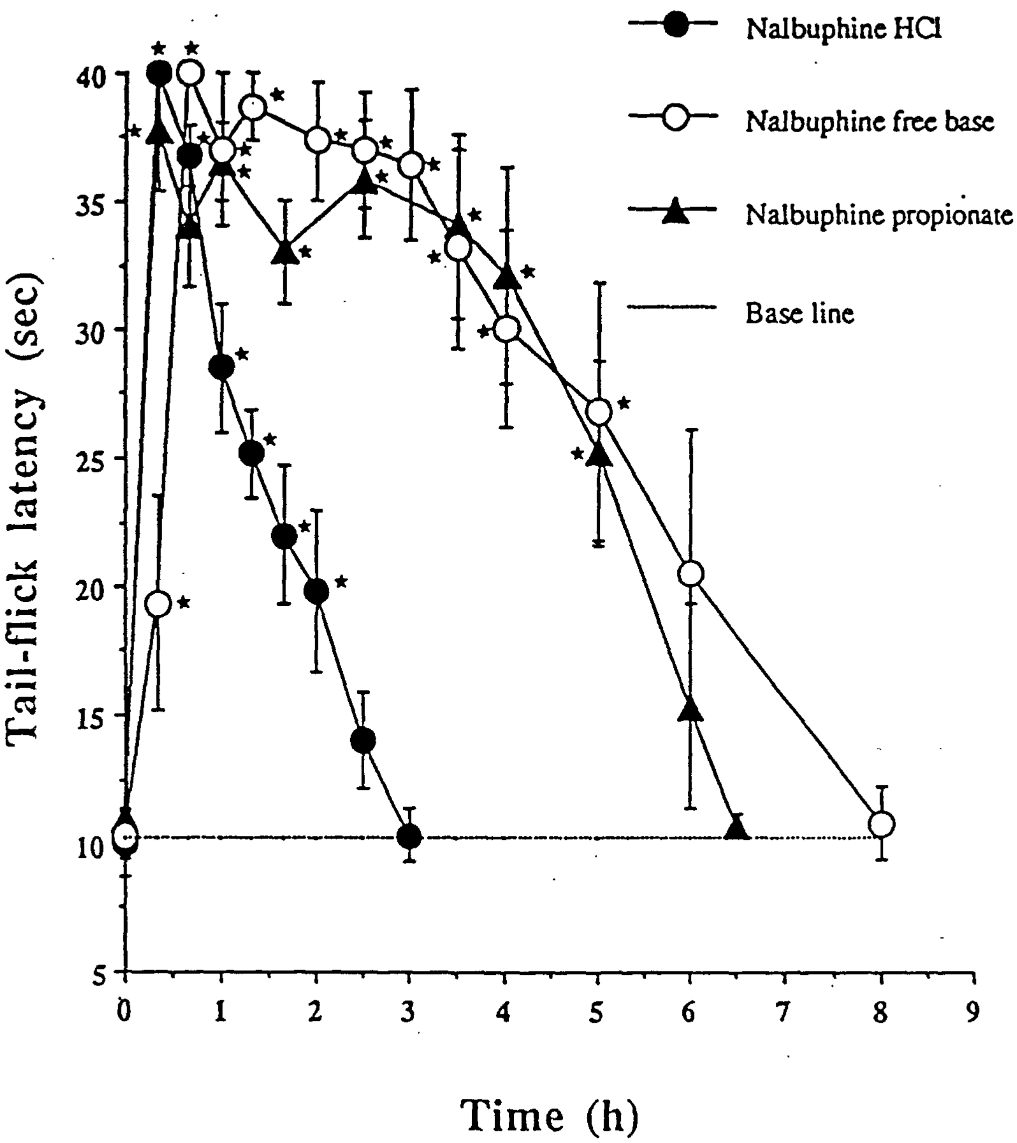


Fig. 18

Example 15 Paw pressure test

(1) animal

Male, New Zealand white rabbits ( 2.4- 2.6 Kg) were used. Each group consisted of 3 rabbits and each was injected once intramuscularly on the long ears.

(2) material

(a) Nalbuphine hydrochloride doses of 20 mg/kg, 10 mg/kg, 5 mg/kg, and 2 mg/kg were used.
(b) Nalbuphine decanoate doses of 0.25 mg/kg were also used.

(3) Experimental

In this experiment, 35, 25, and 15 minutes before dosing, male rabbit paws were tested to measure basic response latency. The time to stop experimentation was set at 50 % press over of the basic latency response before dosing to prevent impairment of health. 5 and 10 minutes after dosing, 1 ml of blood was collected and assayed by a HPLC method.

(4) Results

The maximal analgesic effect can be calculated as follows:

$$\text{The percentage of analgesic effect} = \frac{\left[\begin{array}{l}\text{Latency of}\\\text{experimental}\end{array}\right] - \left[\begin{array}{l}\text{Latency of}\\\text{basic response}\end{array}\right]}{\left[\begin{array}{l}\text{Latency}\\\text{of end}\end{array}\right] - \left[\begin{array}{l}\text{Latency of}\\\text{basic response}\end{array}\right]} \times 100$$

The half time of plasma concentration was found to be 1560 minutes for nalbuphine decanoate, and 51 minutes for nalbuphine hydrochloride. The duration of nalbuphine decanoate was increased 30 times then nalbuphine hydrochloride. (as shown in Figures 19).

The analgesic effect for intramuscular injection was found at 45 minutes for 10 mg/kg (25 mmole/kg) of nalbuphine hydrochloride, and 18 hours for 0.25 mmole/kg of nalbuphine decanoate (as shown in Figures 20).

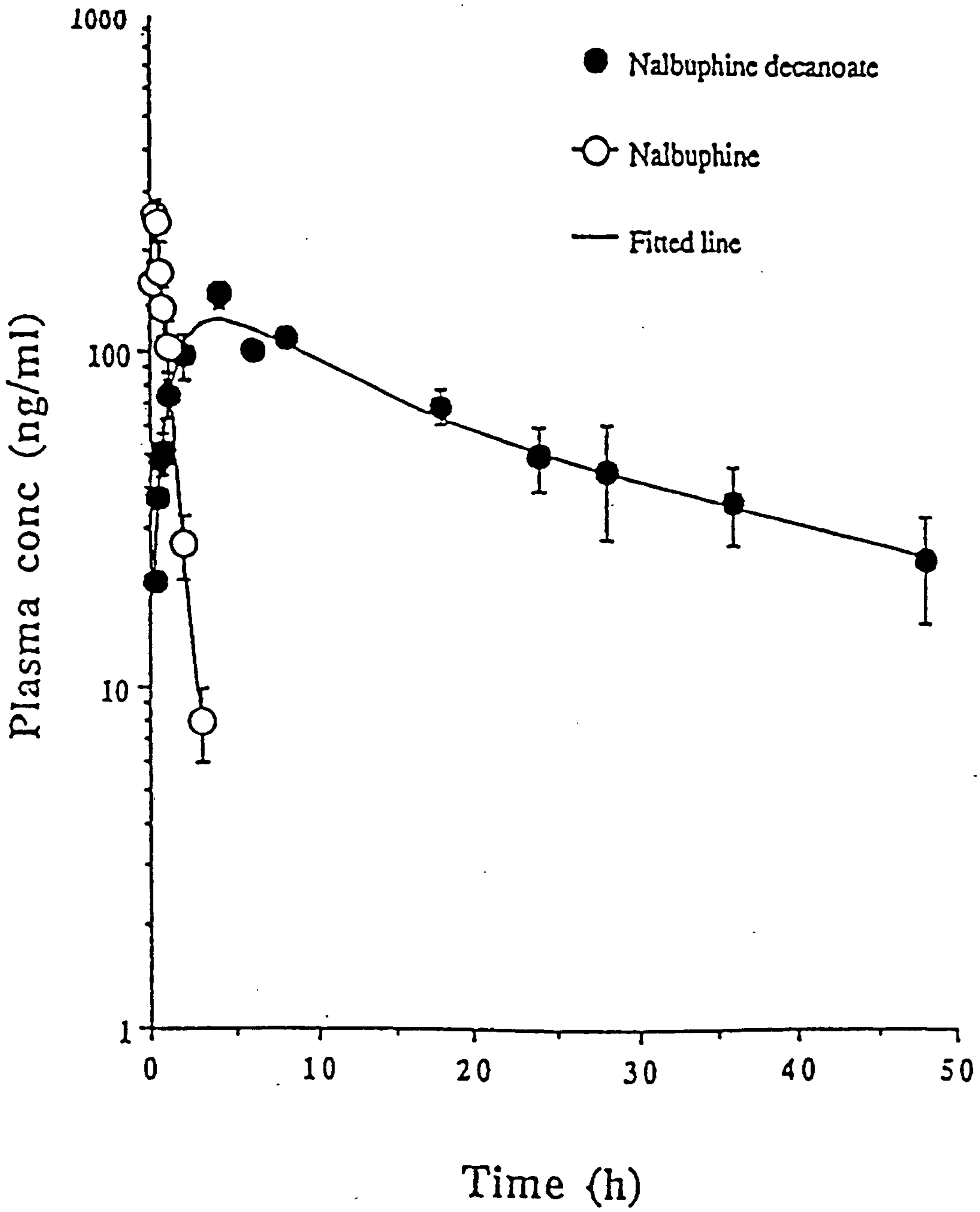
1000
100
10
1
Plasma conc (ng/ml)
Nalbuphine decanoate
Nalbuphine
Fitted line
0
10
20
30
40
50
Time (h)

Fig. 19

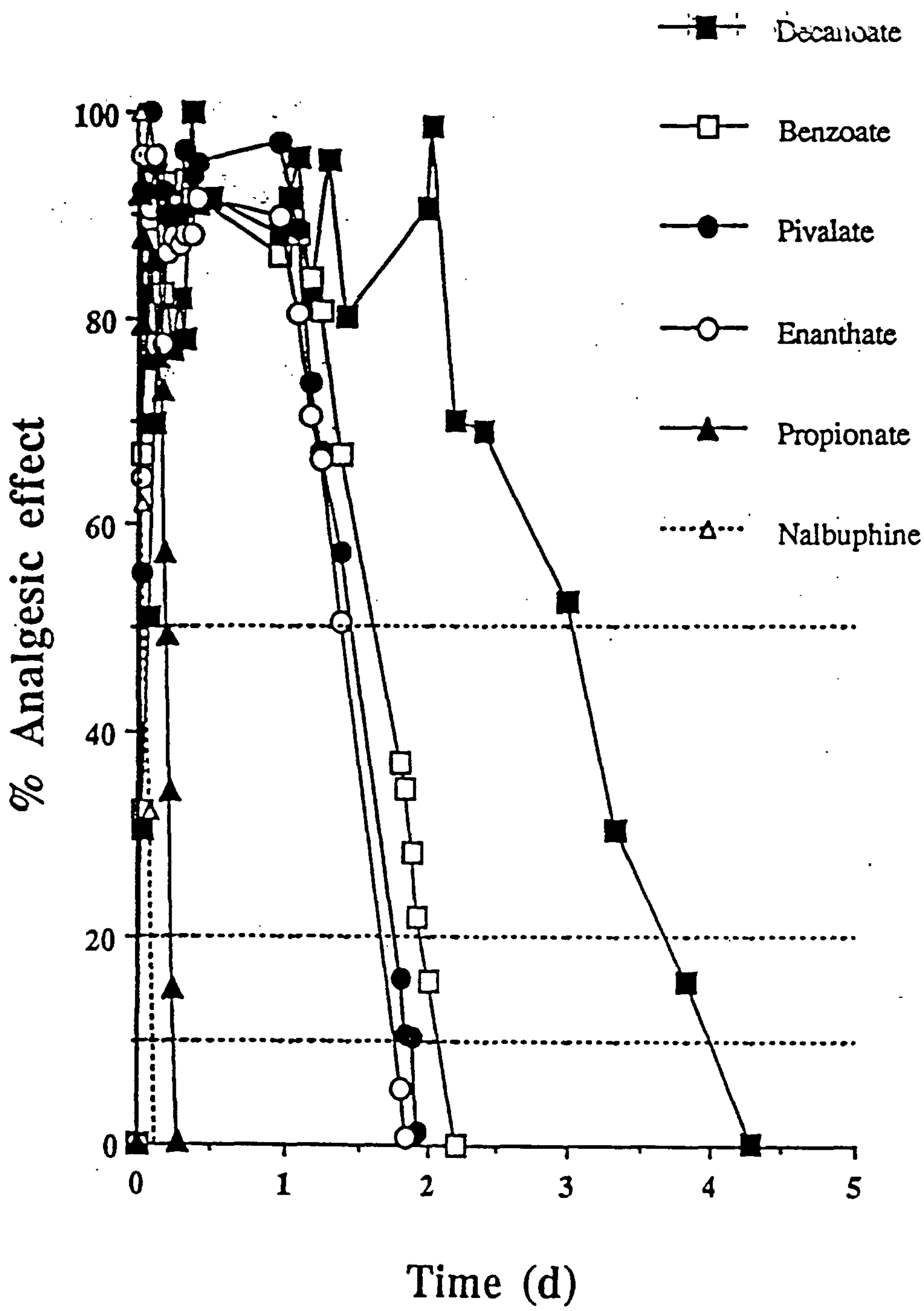


Fig. 20

Example 16 Percutaneous transport study

(1) Animal

Male, New Zealand white rabbits (1 Kg) were used.

(2) material

(a) 5% nalbuphine decanoate in a jel of 2% CMC Na
(b) 5% nalbuphine decanoate in a jel of 2% CMC Na, 10% terpineol.

(3) Experimental

In this experiment, the transdermal penetration of material was measured across full-thickness female rabbit skin *in vitro.* Abdominal skin from New Zealand white rabbits were excised in two pieces, at sacrifice and used immediately. The adherent fat and other visceral debris were removed from the undersurface. The skin with an available diffusion area of 0.72 $cm^2$ separated the donor and receptor chambers of modified Franz diffusion cells which a sampling arm was moved to close the skin to avoid possible air bubble accumulation, also, the bottom of the cell was flattened to smooth the stirring process. The diffusion cells were thermostated at 37 °C throughout the experiment. The receptor compartment of each cell was filled with 5.5 ml of normal saline containing streptomycin sulfate (50 mg/liter) and penicillin G potassium salt (30 mg/liter) and stirred with a magnetic stirrer (600 rpm ). At the start of the experiment a 0.5 g material gel was applied to the exposed epidermal skin surface in the donor phase of the diffusion cell. Subsequently, hourly samples (200 ul) up to 72 hrs. of the receptor perfuse were collected and assayed by HPLC method. The withdraw volume was replaced with fresh medium.

(3) Results

As shown in Figure 21, the typical cumulative amount under 24 hours is 1.04±0.30 μ g/$cm^2$, under 48 hours 1.67±0.38 μg/$cm^2$, and under 72 hours 3.17±0.35 μ g/$cm^2$. The cumulative amount of jell containing 10% terpineol under 24 hours is 3.31±0.45 μg/$cm^2$, under 48 hours 5.78±0.81 μ g/$cm^2$, and under 72 hours 9.87±1.44 μg/$cm^2$.

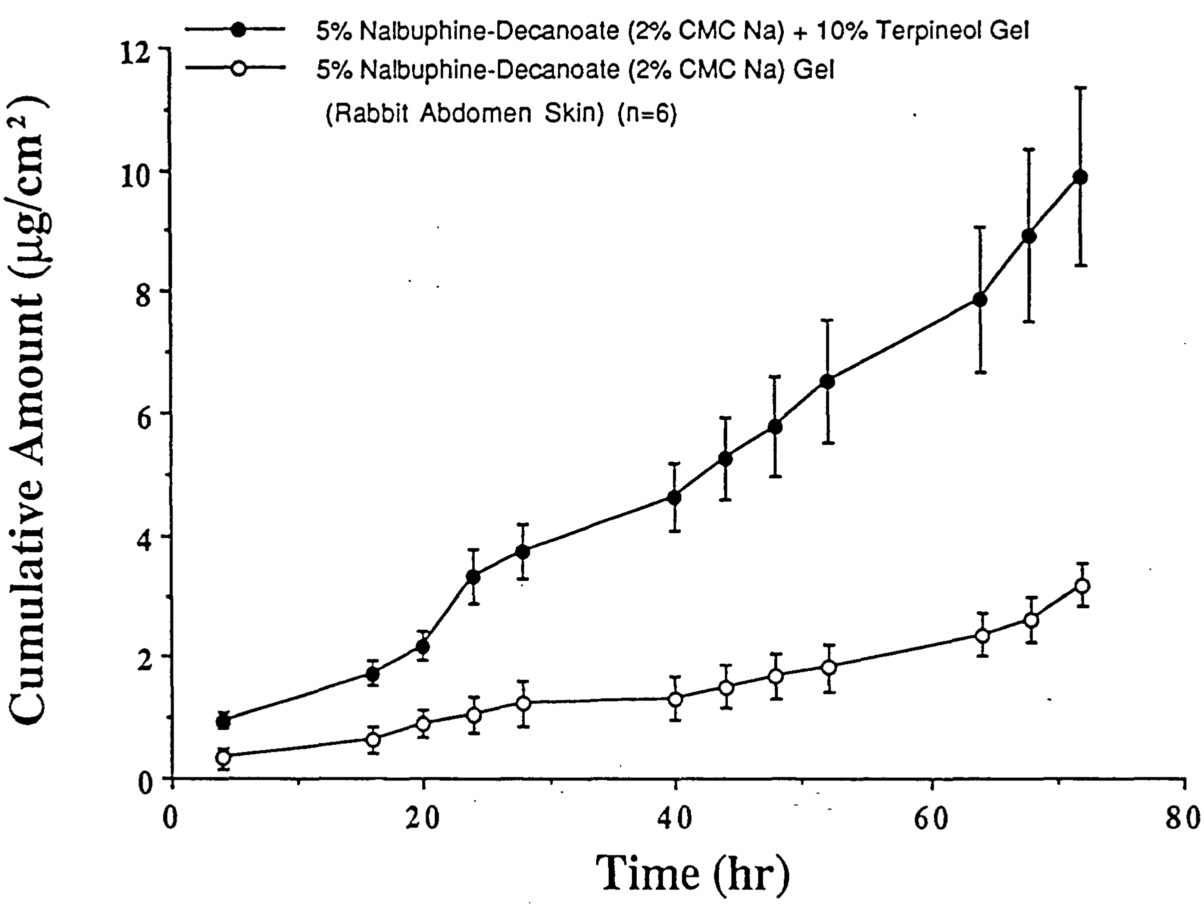


Fig. 21

Tab. 2,

| % Analgesic effect | | | |
|---|---|---|---|
| | 50% | 20% | 10% |
| Nalbuphine HCl | 2.5 H | 4.4 H | 5.0 H |
| N-Decanoate | 6.7 D | 7.5 D | 8.2 D |
| N-Benzoate | 3.5 D | 4.0 D | 4.0 D |
| N-Pivalate | 3.2 D | 3.6 D | 3.8 D |
| N-Enanthate | 3.0 D | 3.4 D | 3.6 D |
| N-Propionate | 10 H | 11 H | 12 H |

## Claims

**1.** A long acting pharmaceutical composition with analgesic activity, which comprises an effective amount of the nalbuphine prodrug or salt of the formula (I)

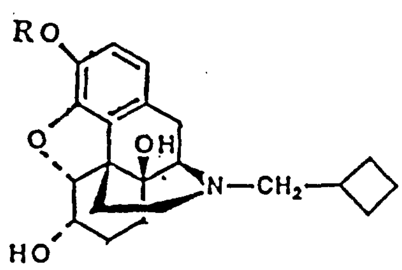

(I)

wherein the R group is R'CO, R' being selected from the groups consisting of (a) straight chain alkyl, (b) branched chain alkyl, (c) straight chain alkyl on benzene, (d) branched chain alkyl on benzene or (e) phenyl, in admixture with a carrier or diluent selected from sesame oil, soybean oil or ethyl ester peanut oil.

**2.** The long acting pharmaceutical composition according to claim 1, wherein R' contains a straight chain saturated or unsaturated C1-40 alkyl group.

**3.** The long acting pharmaceutical composition according to claim 1, wherein R' contains a branched chain saturated or unsaturated C1-40 alkyl group.

**4.** The long acting pharmaceutical composition according to claim 1, wherein R' is a straight chain C6-40 alkyl group on benzene.

**5.** The long acting pharmaceutical composition according to claim 1, wherein R' is a branched chain C6-40 alkyl group on benzene.

**6.** The long acting pharmaceutical composition according to claim 2, wherein the alkyl group is a C1-35 alkyl group.

**7.** The long acting pharmaceutical composition according to claim 3, wherein the alkyl group is a C1-35 alkyl group.

**8.** The long acting pharmaceutical composition according to claim 4, wherein the alkyl group is a C6-35 alkyl group.

**9.** The long acting pharmaceutical composition according to claim 5, wherein the alkyl group is a C6-35 alkyl group.

**10.** The long acting pharmaceutical composition for the treatment of analgesia according to claim 1, which is for administration by injection.

11. A long acting pharmaceutical injection dosage form according to claim 10, for administration by subcutaneous routes.

12. A long acting pharmaceutical injection dosage form according to claim 10, for administration by intramuscular routes.

13. A long acting pharmaceutical injection dosage form according to claim 10, for administration by spinal marrow or intracerebroventricular routes.

14. A long acting pharmaceutical injection dosage form according to claim 10, in which the dosage form is selected from an oily solution injection and oily suspension injection dosage forms.

15. The long acting pharmaceutical composition according to claim 6, wherein the alkyl group is a C1-30 alkyl group.

16. The long acting pharmaceutical composition according to claim 7, wherein the alkyl group is a C1-30 alkyl group.

17. The long acting pharmaceutical composition according to claim 8, wherein the alkyl group is a C6-30 alkyl group.

18. The long acting pharmaceutical composition according to claim 9, wherein the alkyl group is a C6-30 alkyl group.

19. A nalbuphine prodrug or salt of the formula (I)

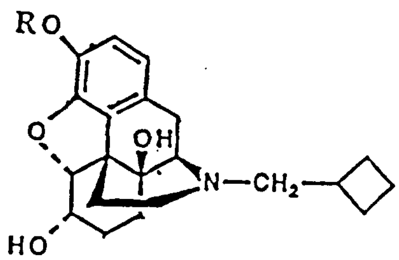

(I)

wherein the R group is R'CO, R'being selected from the groups consisting of (a) C20-40 straight chain alkyl, (b) C20-40 branched chain alkyl, (c) C26-40 straight chain alkyl on benzene or (d) C26-40 branched chain alkyl on benzene.

20. A nalbuphine prodrug or salt according to claim 19, wherein R' is a straight chain saturated or unsaturated C20-40 alkyl group.

21. A nalbuphine prodrug or salt, according to claim 19, wherein R' is a branched chain saturated or unsaturated C20-40 alkyl group.

22. A nalbuphine prodrug or salt according to claim 19, wherein R' is a straight chain saturated or unsaturated C26-40 alkyl group on benzene.

23. A nalbuphine prodrug or salt according to claim 19,-wherein R' is a branched chain saturated or unsaturated C26-40 alkyl group on benzene.

24. A nalbuphine prodrug or salt- according to claim 20, wherein R' is a saturated or unsaturated C30-40 alkyl group.

25. A nalbuphine prodrug or salt according to claim 21, wherein R' is a saturated or unsaturated C30-40 alkyl group.

26. A process for the preparation of nalbuphine prodrugs and salts of claim 19, which comprises reacting nalbuphine with methylene chloride and a compound of the formula (II):

R'-COOH (II)

wherein R' is selected from the groups consisting of (a) C20-40 straight chain alkyl, (b) C20-40 branched chain alkyl, (c) C26-40 straight chain alkyl on benzene, or (d) C26-40 branched chain alkyl on benzene.

**27.** A process according to claim 26, wherein R' is a straight chain saturated or unsaturated C20-40 alkyl group.

**28.** The long acting pharmaceutical composition according to claim 15, wherein the alkyl group is a C1-25 alkyl group.

**29.** The long acting pharmaceutical composition according to claim 16, wherein the alkyl group is a C1-25 alkyl group.

**30.** The long acting pharmaceutical composition according to claim 17, wherein the alkyl group is a C6-25 alkyl group.

**31.** The long acting pharmaceutical composition according to claim 18, wherein the alkyl group is a C6-25 alkyl group.

**32.** Nalbuphine decanoate and its salts.

**33.** Nalbuphine enanthate and its salts.

**34.** Nalbuphine behenate and its salts.

**35.** Nalbuphine erucicate and its salts.

**36.** Nalbuphine arachidate and its salts.

**37.** A process according to claim 26, wherein R' is a branched chain saturated or unsaturated C20-40 alkyl group.

**38.** A process according to claim 26, wherein R' is a straight chain saturated or unsaturated C26-40 alkyl group on benzene.

**39.** A process according to claim 26, wherein R' is a branched chain saturated or unsaturated C26-40 alkyl group on benzene.

**40.** The long acting pharmaceutical composition for the treatment of analgesia according to claim 1, comprising a pharmaceutically acceptable carrier or diluent for percutaneous administration.

**Patentansprüche**

**1.** Langzeitarzneimittel mit schmerzlindernder Aktivität, die eine wirksame Menge der Nalbuphinprodroge oder -salz der Formel

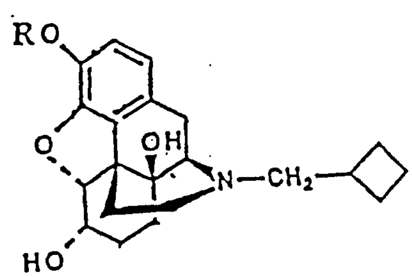

(I)

aufweist, in der die R-Gruppe R'CO ist, wobei R' von den Gruppen ausgewählt ist, die aus (a) geradkettigem Alkyl, (b) Alkyl mit verzweigter Kette, (c) geradkettigem Alkyl auf Benzen, (d) Alkyl mit verzweigter Kette auf Benzen oder (e) Phenyl bestehen, in Zumischung mit einem Träger oder einem Verdünnungsmittel, das von Sesamöl,

Soyabohnenöl, oder Ethylestererdnussöl ausgewählt ist.

**2.** Langzeitarzneimittel nach Anspruch 1, in dem R' eine geradkettige gesättigte oder ungesättigte C1-40-Alkylgruppe enthält.

**3.** Langzeitarzneimittel nach Anspruch 1, in dem R' eine gesättigte oder ungesättigte Cl-40-Alkylgruppe mit verzweigter Kette enthält.

**4.** Langzeitarzneimittel nach Anspruch 1, in dem R' eine geradkettige C6-40-Alkylgruppe auf Benzen ist.

**5.** Langzeitarzneimittel nach Anspruch 1, in dem R' eine C6-40-Alkylgruppe mit verzweigter Kette auf Benzen ist.

**6.** Langzeitarzneimittel nach Anspruch 2, in dem die Alkylgruppe eine C1-35-Alkylgruppe ist.

**7.** Langzeitarzneimittel nach Anspruch 3, in dem die Alkylgruppe eine C1-35-Alkylgruppe ist.

**8.** Langzeitarzneimittel nach Anspruch 4, in dem die Alkylgruppe eine C6-35-Alkylgruppe ist.

**9.** Langzeitarzneimittel nach Anspruch 5, in dem die Alkylgruppe eine C6-35-Alkylgruppe ist.

**10.** Langzeitarzneimittel zur Behandlung von Schmerzen nach Anspruch 1, die zur Verabreichung durch Einspritzung ist.

**11.** Langzeitarzneieinspritzungsdosierungsform nach Anspruch 10 zur Verabreichung durch subkutane Wege.

**12.** Langzeitarzneieinspritzungsdosierungsform nach Anspruch 10 zur Verabreichung durch intramuskulare Wege.

**13.** Langzeitarzneieinspritzungsdosierungsform nach Anspruch 10 zur Verabreichung durch Rückenmark- oder intracerebroventriculare Wege.

**14.** Langzeitarzneieinspritzungsdosierungsform nach Anspruch 10, in der die Dosierungsform von öligen Lösungseinspritzungs- und öligen Suspensionseinspritzungsdosierungsformen ausgewählt ist.

**15.** Langzeitarzneimittel nach Anspruch 6, in der die Alkylgruppe eine C1-30-Alkylgruppe ist.

**16.** Langzeitarzneimittel nach Anspruch 7, in der die Alkylgruppe eine C1-30-Alkylgruppe ist.

**17.** Langzeitarzneimittel nach Anspruch 8, in der die Alkylgruppe eine C6-30-Alkylgruppe ist.

**18.** Langzeitarzneimittel nach Anspruch 9, in der die Alkylgruppe eine C6-30-Alkylgruppe ist.

**19.** Nalbuphinprodroge oder -salz der Formel (I)

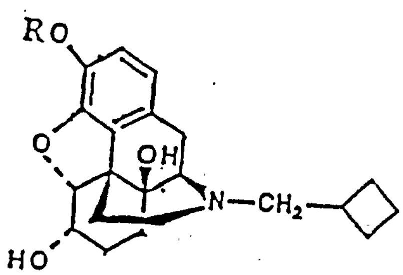

(I)

in der die R-Gruppe R'CO ist, wobei R' von der Gruppe ausgewählt ist, die aus (a) geradkettigem C20-40-Alkyl, (b) C20-40-Alkyl mit verzweigter Kette, (c) geradkettigem C20-40-Alkyl auf Benzen, (d) C20-40-Alkyl mit verzweigter Kette auf Benzen.

20. Nalbuphinprodroge oder -salz nach Anspruch 19, in der R' eine geradkettige gesättigte oder ungesättigte C20-40-Alkylgruppe ist.

21. Nalbuphinprodroge oder -salz nach Anspruch 19, in der R' eine gesättigte oder ungesättigte C20-40-Alkylgruppe mit verzweigter Kette ist.

22. Nalbuphinprodroge oder -salz nach Anspruch 19, in der R' eine geradkettige gesättigte oder ungesättigte C26-40-Alkylgruppe auf Benzen ist.

23. Nalbuphinprodroge oder -salz nach Anspruch 19, in der R' eine gesättigte oder ungesättigte C20-40-Alkylgruppe mit verzweigter Kette auf Benzen ist.

24. Nalbuphinprodroge oder -salz nach Anspruch 20, in der R' eine gesättigte oder ungesättigte C30-40-Alkylgruppe ist.

25. Nalbuphinprodroge oder -salz nach Anspruch 21, in der R' eine gesättigte oder ungesättigte C30-40-Alkylgruppe ist.

26. Verfahren zur Herstellung von Nalbuphinprodrogen und -salzen nach Anspruch 19, das aufweist, Nalbuphin mit Methylenchlorid und einer Zusammensetzung der Formel (II):

$$R'COOH \qquad (II)$$

zu reagieren, worin R' von den Gruppen ausgewählt ist, die aus (a) geradkettigem C20-40-Alkyl, (b) C20-40-Alkyl mit verzweigter Kette, (c) geradkettigem C20-40-Alkyl auf Benzen, (d) C20-40-Alkyl mit verzweigter Kette auf Benzen bestehen.

27. Verfahren nach Anspruch 26, in dem R' eine geradkettigte gesättigte oder ungesättigte C20-40-Alkylgruppe ist.

28. Langzeitarzneimittel nach Anspruch 15, in dem die Alkylgruppe eine C1-25-Alkylgruppe ist.

29. Langzeitarzneimittel nach Anspruch 16, in dem die Alkylgruppe eine C1-25-Alkylgruppe ist.

30. Langzeitarzneimittel nach Anspruch 17, in dem die Alkylgruppe eine C6-25-Alkylgruppe ist.

31. Langzeitarzneimittel nach Anspruch 18, in dem die Alkylgruppe eine C6-25-Alkylgruppe ist.

32. Nalbuphindecanoat und seine Salze.

33. Nalbuphinenanthat und seine Salze.

34. Nalbuphinbehenat und seine Salze.

35. Nalbuphinerucicat und seine Salze.

36. Nalbuphinarachidat und seine Salze.

37. Verfahren nach Anspruch 26, in dem R' eine gesättigte oder ungesättigte C20-40-Alkylgruppe mit verzweigter Kette ist.

38. Verfahren nach Anspruch 26, in dem R' eine geradkettige gesättigte oder ungesättigte C20-40-Alkylgruppe auf Benzen ist.

39. Verfahren nach Anspruch 26, in dem R' eine gesättigte oder ungesättigte C20-40-Alkylgruppe mit verzweigter Kette auf Benzen ist.

40. Langzeitarzneimittel zur Behandlung von Schmerzen nach Anspruch 1, die einen pharmazeutisch annehmbaren

Träger oder ein Verdünnungsmittel für perkutane Verabreichung aufweist.

**Revendications**

1. Composé pharmaceutique de longue durée de fonction ayant un effet analgésique, comportant un montant effectif du produit précurseur de nalbuphine ou du sel selon la formule (I)

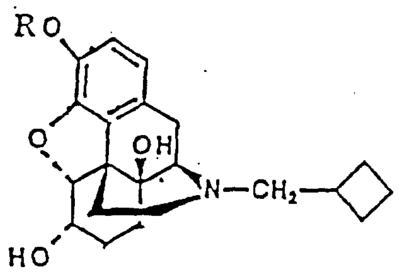

(I)

dont le groupe R est R'CO', R' ayant été sélectionné à partir des groupes comportant (a) l'alcoyle à chaîne linéaire, (b) l'alcoyle à chaîne ramifiée, (c) l'alcoyle à chaîne linéaire sur la benzène, (d) l'alcoyle à chaîne ramifiée sur la benzène, ou (e) le phényle, en matière ajoutée avec un support ou diluant sélectionné à partir d'huile de sésame, d'huile de soja, ou d'huile éthylique ester de cacahuète.

2. Le composé pharmaceutique de longue durée de fonction selon la revendication 1, dont R' contient un groupe alcoyle C1-40 à chaîne linéaire saturée ou non-saturée.

3. Le composé pharmaceutique de longue durée de fonction selon la revendication 1, dont R' contient un groupe alcoyle C1-40 à chaîne ramifiée saturée ou non-saturée.

4. Le composé pharmaceutique de longue durée de fonction selon la revendication 1, dont R' est un groupe alcoyle C6-40 à chaîne linéaire sur la benzène.

5. Le composé pharmaceutique de longue durée de fonction selon la revendication 1, dont R' est un groupe alcoyle C6-40 à chaîne ramifiée sur la benzène.

6. Le composé pharmaceutique de longue durée de fonction selon la revendication 2, dont le groupe alcoyle est un groupe alcoyle C1-35.

7. Le composé pharmaceutique de longue durée de fonction selon la revendication 3, dont le groupe alcoyle est un groupe alcoyle C1-35.

8. Le composé pharmaceutique de longue durée de fonction selon la revendication 4, dont le groupe alcoyle est un groupe alcoyle C6-35.

9. Le composé pharmaceutique de longue durée de fonction selon la revendication 5, dont le groupe alcoyle est un groupe alcoyle C6-35.

10. Le composé pharmaceutique de longue durée de fonction pour le traitement analgésique selon la revendication 1, qui est prévu pour administrer par injection.

11. La forme de posologie par injection de produit pharmaceutique de longue durée de fonction selon la revendication 10, pour administrer par les voies sous-cutanées.

12. La forme de posologie de produit pharmaceutique de longue durée de fonction selon la revendication 10, pour administrer par les voies intramusculaires.

13. La forme de posologie par injection de produit pharmaceutique de longue durée de fonction selon la revendication

10, pour administrer par les voies de la moelle épinière ou intracérébroventriculaire.

**14.** La forme de posologie de produit pharmaceutique de longue durée de fonction selon la revendication 10, dont ladite forme de posologie est sélectionnée à partir des formes de posologie pour l'injection en solution oléagineuse et l'injection en suspension oléagineuse.

**15.** Le composé pharmaceutique de longue durée de fonction selon la revendication 6, dont le groupe alcoyle est un groupe alcoyle Cl-30.

**16.** Le composé pharmaceutique de longue durée de fonction selon la revendication 7, dont le groupe alcoyle est un groupe alcoyle C1-30.

**17.** Le composé pharmaceutique de longue durée de fonction selon la revendication 8, dont le groupe alcoyle est un groupe alcoyle C6-30.

**18.** Le composé pharmaceutique de longue durée de fonction selon la revendication 9, dont le groupe alcoyle est un groupe alcoyle C6-30.

**19.** Produit précurseur de nalbuphine ou du sel selon la formule (I)

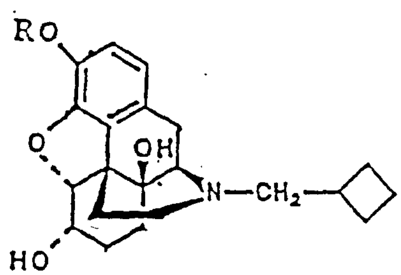

(I)

dont le groupe R est R'CO', R' ayant été sélectionné à partir des groupes comportant (a) l'alcoyle à chaîne linéaire C20-40, (b) l'alcoyle à chaîne ramifiée C20-40, (c) l'alcoyle à chaîne linéaire C26-40 sur benzène, ou (d) l'alcoyle à chaîne ramifiée C26-40 sur benzène.

**20.** Produit précurseur ou sel de nalbuphine selon la revendication 19, dont R' est un groupe à chaîne linéaire alcoyle C20-40 saturée ou non-saturée.

**21.** Produit précurseur ou sel de nalbuphine selon la revendication 19, dont R' est un groupe à chaîne ramifiée alcoyle C20-40 saturée ou non-saturée.

**22.** Produit précurseur ou sel de nalbuphine selon la revendication 19, dont R' est un groupe à chaîne linéaire alcoyle C26-40 saturée ou non-saturée sur benzène.

**23.** Produit précurseur ou sel de nalbuphine selon la revendication 19, dont R' est un groupe alcoyle à chaîne ramifiée C26-40 saturée ou non-saturée sur benzène.

**24.** Produit précurseur ou sel de nalbuphine selon la revendication 20, dont R' est un groupe saturé ou non-saturé alcoyle C30-40 .

**25.** Produit précurseur ou sel de nalbuphine selon la revendication 21, dont R' est un groupe saturé ou non-saturé alcoyle C30-40 .

**26.** Procédé de préparation de produits précurseurs ou de sels de nalbuphine à la revendication 19, qui comporte une réaction de nalbuphine avec le chlorure de méthylène et un composé de formule (II):

$$R'\text{-}COOH \qquad (II)$$

dont R' est sélectionné à partir des groupes comportant (a) l'alcoyle à chaîne linéaire C20-40, (b) l'alcoyle à chaîne ramifiée C20-40, (c) l'alcoyle à chaîne linéaire C26-40 sur benzène, ou (d) l'alcoyle à chaîne ramifiée C26-40 sur benzène.

**27.** Procédé à la revendication 26, dont R' est un groupe alcoyle à chaîne linéaire C20-40 saturée ou non-saturée.

**28.** Le composé pharmaceutique de longue durée de fonction selon la revendication 15, dont le groupe alcoyle est un groupe alcoyle C1-25.

**29.** Le composé pharmaceutique de longue durée de fonction selon la revendication 16, dont le groupe alcoyle est un groupe alcoyle C1-25.

**30.** Le composé pharmaceutique de longue durée de fonction selon la revendication 17, dont le groupe alcoyle est un groupe alcoyle C6-25.

**31.** Le composé pharmaceutique de longue durée de fonction selon la revendication 18, dont le groupe alcoyle est un groupe alcoyle C6-25.

**32.** Le décoanate de nalbuphine et ses sels.

**33.** L'énanthate de nalbuphine et ses sels.

**34.** Le béhénate de nalbuphine et ses sels.

**35.** L'érucicate de nalbuphine et ses sels.

**36.** L'arachidate de nalbuphine et ses sels.

**37.** Procédé selon la revendication 26, dont R' est un groupe alcoyle C20-40 à chaîne ramifiée saturée ou non-saturée.

**38.** Procédé selon la revenndication 26, dont R' est un groupe alcoyle C26-40 à chaîne linéaire saturée ou non-saturée sur benzène.

**39.** Procédé selon la revenndication 26, dont R' est un groupe alcoyle C26-40 à chaîne ramifiée saturée ou non-saturée sur benzène.

**40.** Le composé pharmaceutique de longue durée de fonction de l'analgésie selon la revendication 1, comportant un porteur ou diluant admis an niveau pharmaceutique pour l'administration percutanée.